(19) Europäisches Patentamt
European Patent Office
Office européen des brevets

(11) Veröffentlichungsnummer : **0 088 346**
**B1**

(12) # EUROPÄISCHE PATENTSCHRIFT

(45) Veröffentlichungstag der Patentschrift : **05.06.85**

(21) Anmeldenummer : **83102005.2**

(22) Anmeldetag : **02.03.83**

(51) Int. Cl.⁴ : **C 07 C147/14**, C 07 C147/12, C 07 D295/08, A 61 K 31/135, C 07 C147/10

(54) **2-Aminomethyl-phenol-Derivate, Verfahren zu ihrer Herstellung, ihre Verwendung sowie pharmazeutische Präparate auf Basis dieser Verbindungen.**

(30) Priorität : **06.03.82 DE 3208190**

(43) Veröffentlichungstag der Anmeldung : **14.09.83 Patentblatt 83/37**

(45) Bekanntmachung des Hinweises auf die Patenterteilung : **05.06.85 Patentblatt 85/23**

(84) Benannte Vertragsstaaten : **AT BE CH DE FR GB IT LI LU NL SE**

(56) Entgegenhaltungen :
**FR-A- 1 588 892**
**US-A- 3 954 871**
**JOURNAL OF MEDICINAL CHEMISTRY, Band 23, Nr. 12, 1980 G.E. STOKKER et al.: "2-(amino-methyl) phenols, a new class of saluretic agents. 1. Effects of nuclear substitution.", Seiten 1414-1427**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Englert, Heinrich Christian, Dr.**
**Wielandstrasse 6**
**D-6238 Hofheim am Taunus (DE)**
Erfinder : **Lang, Hans-Jochen, Dr.**
**Rüdesheimer Strasse 7**
**D-6238 Hofheim am Taunus (DE)**
Erfinder : **Hropot, Max, Dr.**
**Friedrich-Stolz-Strasse 13**
**D-6093 Flörsheim am Main (DE)**
Erfinder : **Kaiser, Joachim, Dr.**
**Fichtestrasse 12**
**D-6000 Frankfurt am Main (DE)**

**EP 0 088 346 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 2-Aminomethyl-phenole der Formel I

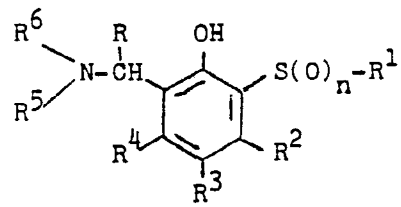

(I)

in welcher

R für Wasserstoff oder Alkyl mit 1 oder 2 C-Atomen,

$R^1$ für Alkyl mit 1 bis 8 C-Atomen, Cycloalkyl mit 3 bis 12 C-Atomen und bis zu 8 Ringgliedern, Alkenyl mit 2 bis 8 C-Atomen oder Cycloalkenyl mit 5 bis 12 C-Atomen und bis zu 8 Ringgliedern steht, wobei jeder der vorstehend genannten Reste mit 1 bis 5 gleichen oder verschiedenen Halogenatome substituiert sein kann,

$R^2$ und $R^4$ gleich oder verschieden sind und Wasserstoff, Halogen, Alkyl mit 1 oder 2 C-Atomen oder Alkoxy mit 1 oder 2 C-Atomen

$R^3$ Halogen, Alkyl mit 1 bis 12 C-Atomen oder Cycloalkyl mit 3 bis 12 C-Atomen und bis zu 8 Ringgliedern bedeuten,

$R^5$ und $R^6$ gleich oder verschieden sind und für Wasserstoff, Alkyl mit 1 bis 6 C-Atomen, Cycloalkyl mit 3 bis 10 C-Atomen und bis zu 8 Ringgliedern oder den Rest $-[CH_2]_o-Ar$, worin o = 1 oder 2 ist, die $-[CH_2]_o-$ Kette durch 1 oder 2 ($C_1$ oder $C_2$)-Alkylgruppen substituiert sein kann und Ar ein gegebenenfalls durch 1 bis 3 gleiche oder verschiedene Reste ($C_1$ oder $C_2$)-Alkyl, ($C_1$ oder $C_2$)-Alkoxy oder Halogen substituiertes, 5- oder 6-gliedriges aromatisches oder heteroaromatisches System ist, stehen und n 1 oder 2 ist, wobei die Reste $R^5$ und $R^6$ und/oder zwei der Reste $P^2$, $R^3$ und $R^4$ auch eine $-[CH_2]_m-$ Kette mit m = 3 bis 6 bilden können, die gegebenenfalls durch 1 oder 2 Methylgruppen substituiert und die im Falle von $R^5$ und $R^6$ auch durch 1 oder 2 Methylgruppen substituiert und die im Falle von $R^5$ und $R^6$ auch durch 1 oder 2 Sauerstoffatome, Schwefelatome und/oder Iminogruppen unterbrochen sein kann sowie deren physiologisch verträglichen Salze.

Unter einem aromatischen System Ar wird vorzugsweise Phenyl verstanden. Ein 5- oder 6-gliedriges heteroaromatisches System Ar ist vorzugsweise ein Rest eines 5- oder 6- gliedriger O-, N- und/oder S-heterocyclischen Ringes, insbesondere Furyl, Thienyl, Pyrrolyl, Thiazolyl, Isothiazolyl, Oxazolyl, Isoxazolyl, Pyrazolyl, Imidazolyl, Pyridyl, Pyrazinyl, Pyrimidinyl, Pyridazinyl, Triazinyl. Unter Halogen als Substituent an Ar wird F, Cl, Br und/oder J, vorzugsweise F und/oder Cl verstanden.

Hat eine der vorstehend definierten Cycloalkyl- oder Cycloalkenylgruppen eine C-Zahl, die über die Zahl der Ringglieder hinausgeht, so wird darunter eine Cycloalkyl- bzw. Cycloalkenylgruppe verstanden, die durch eine oder mehrere Alkylgruppen substituiert ist oder eine ggf. alkylsubstituierte Cycloalkylalkyl- bzw. Cycloalkenylalkylgruppe.

Falls Verbindungen der Formel I chirale C- und/oder S-Atome aufweisen, so sind sowohl am jeweiligen Zentrum S-konfigurierte als auch R-konfigurierte Verbindungen Gegenstand der Erfindung. Die Verbindungen können dann als optische Isomere, als Diastereomere, als Racemate oder als Gemische derselben vorliegen.

Aus J. med. Chem. *23* [1980], Seite 1414-1427 sind in 6-Stellung substituierte 2-Aminomethyl-phenole bekannt. Die 4-Alkyl-6-halogen-Derivate haben salidiuretische Eigenschaften von Art und Stärke, wie es kurz und intensiv wirksame Diuretika, wie beispielsweise Furosemid, zeigen. Speziell bei einem 6-Jod-Derivat wird auch eine antihypertensive Zusatzwirkung beobachtet.

Es war daher äußerst überraschend, daß die erfindungsgemäßen Verbindungen der Formel I an Hund oder Ratte eine salidiuretische Wirkung ausüben, die die Größenordnung der Aktivität der oben beschriebenen Halogenderivate, insbesondere der Jodverbindung erreichen können. Sie unterscheiden sich jedoch von diesen vorteilhaft dadurch, daß sie eine wesentlich geringere akute Toxizität aufweisen, wie dies an Ratten aufgezeigt werden kann.

Weiterhin war es überraschend, daß einige der erfindungsgemäßen Verbindungen nicht nur eine sehr gute salidiuretische Wirksamkeit aufweisen, sondern auch an spontan hypertensiven Ratten in Dosen unter 20 mg/kg stark blutdrucksenkend wirken.

Bevorzugt sind Verbindungen der Formel I, die dadurch gekennzeichnet sind, daß R für Wasserstoff,

$R^1$ für Alkyl mit 1 bis 4 C-Atomen, Cycloalkyl mit 3 bis 7 C-Atomen und bis zu 6 Ringgliedern, Alkenyl mit 2 bis 6 C-Atomen oder Halogenalkyl mit 1 bis 4 C-Atomen und 1 bis 3 gleichen oder verschiedenen Halogenatomen steht,

$R^3$ Halogen, Alkyl mit 1 bis 8 C-Atomen oder Cycloalkyl mit 3 bis 10 C-Atomen und bis zu 7 Ringgliedern bedeutet

$R^5$ und $R^6$ die vorstehend definierten Bedeutungen haben mit der Ausnahme, daß $R^5$ und/oder $R^6$ für Alkyl mit 1 oder 2 C-Atomen steht

n 1 oder 2 ist, wobei die Reste $R^5$ und $R^6$ und/oder zwei der Reste $R^2$, $R^3$ und $R^4$ eine wie vorstehend definierte Polymethylenkette bilden können.

Insbesondere kommen von den Verbindungen der Formel I diejenigen in Betracht, in denen

R Wasserstoff,

$R^1$ Methyl, Chlormethyl, Jodmethyl oder Ethyl,

$R^2$ und $R^4$ Wasserstoff, $R^3$ iso-Propyl, tert.-Butyl, tert.-Amyl oder sec. Butyl, $R^5$ und $R^6$ Wasserstoff und

n 1 oder 2 bedeuten, wobei als besonders bevorzugte Verbindungen solche hervorzuheben sind, bei denen

R für Wasserstoff

$R^1$ für Methyl oder Chlormethyl, $R^2$ und $R^4$ für Wasserstoff,

$R^3$ für tert. Butyl, $R^5$ und $R^6$ für Wasserstoff und n für 2 steht.

Die Erfindung betrifft weiterhin ein Verfahren zur Herstellung von 2-Aminomethyl-phenolen der Formel I, das dadurch gekennzeichnet ist, daß man

a) Verbindungen der Formel II

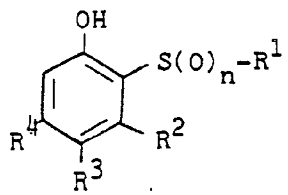

(II)

worin n, $R^1$, $R^2$, $R^3$ und $R^4$ die oben genannten Bedeutungen besitzen, mit einem N-Hydroxymethylcarboxamid der allgemeinen Formel III,

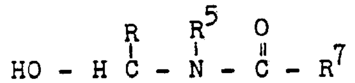

(III)

in der R Wasserstoff oder Alkyl mit 1 oder 2 C-Atomen, $R^5$ Wasserstoff oder Alkyl mit 1 bis 4 C-Atomen bedeutet und $R^7$ für Wasserstoff, gegebenenfalls halogensubstituiertes Alkyl mit 1 bis 4 C-Atomen oder Aryl mit 6 bis 10 C-Atomen steht oder in der $R^5$ und der Rest $COR^7$ gemeinsam für den o-Phthaloylrest stehen, zu Verbindungen der allgemeinen Formel IV umsetzt

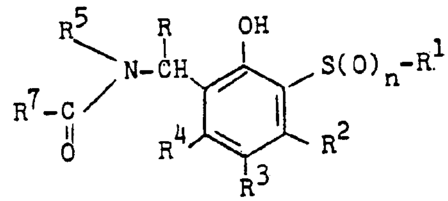

(IV)

und den Acylrest $R^7$—CO hydrolytisch abspaltet oder

b) Verbindungen der Formel II, in der die Reste $R^1$ bis $R^4$ und n die oben erwähnten Bedeutungen haben, in Gegenwart von Formaldehyd mit Aminen der allgemeinen Formel V,

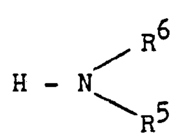

(V)

in der $R^5$ und $R^6$ die oben genannten Bedeutungen mit Ausnahme der von Wasserstoff besitzen, umsetzt,

c) Verbindungen der allgemeinen Formel VI,

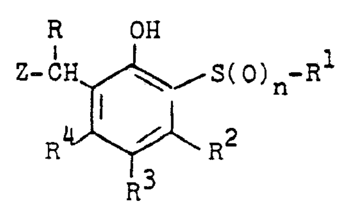

(VI)

in der n, R bis $R^4$ die oben genannten Bedeutungen haben und Z für eine Fluchtgruppe steht mit Aminen der allgemeinen Formel H—N = Y, wobei Y für eine Aminschutzgruppe oder für die in Anspruch 1 definierten Reste $R^5$ und $R^6$ steht, wobei $R^6$ auch eine Aminschutzgruppe sein kann, zu Verbindungen der allgemeinen Formel VII umsetzt

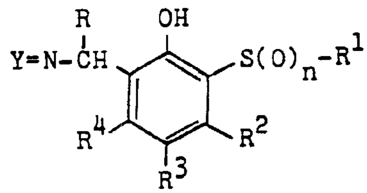

(VII)

und gegebenenfalls die Aminschutzgruppe hydrolytisch oder hydrogenolytisch abspaltet oder

d) Verbindungen der allgemeinen Formel XIV

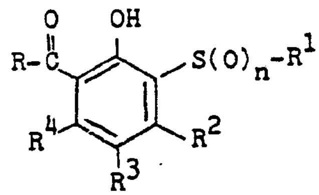

(XIV)

in der n und R bis $R^4$ die oben genannten Bedeutungen haben, umsetzt mit Aminen der Formel $R^5$—$NH_2$ mit $R^5$ in der oben genannten Bedeutung zu Schiff'schen Basen der Formel XV

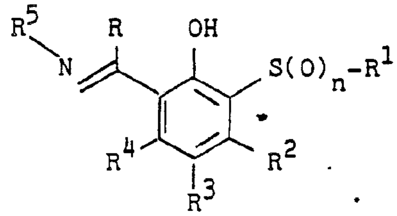

(XV)

in der n und R bis $R^5$ die oben genannten Bedeutungen haben und diese zu Verbindungen der Formel I reduziert, und die nach einer der Varianten a)-d) erhaltenen Verbindungen der Formel I gegebenenfalls in ihre physiologisch verträglichen Salze überführt.

Als Fluchtgruppen Z der Verfahrensvariante c sind alle leicht nucleophil verdrängbaren Gruppen, wie beispielsweise Halogen, Tosyl, Dimethylamino oder Trimethylammonium, geeignet. Y als Aminoschutzgruppe bedeutet beispielsweise den Diazorest oder den Phthaloylrest. $R^6$ als Aminoschutzgruppe steht für die aus Peptidsynthese bekannten einwertigen Reste, wie den Benzylrest oder andere Acylreste —CO—$R^7$, wobei $R^7$ die vorstehend genannte Bedeutung zukommt.

Die Erfindung betrifft weiterhin Verbindungen der allgemeinen Formel II, in welcher n, $R_1$ bis $R^4$ die vorstehend genannte Bedeutung haben. Sie sind Vorprodukte bei der Herstellung von Verbindungen der allgemeinen Formel I.

Die Abspaltung des Restes

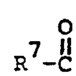

von Verbindungen der allgemeinen Formel IV gemäß Verfahrensweise a) wird im allgemeinen mit Hilfe einer Säure oder einer Base in Gegenwart von Wasser vorgenommen. Erfolgt die Abspaltung unter sauren Bedingungen, so findet vorzugsweise eine starke Mineralsäure wie Salzsäure, Brom- oder Jodwasserstoffsäure oder Schwefelsäure Verwendung. Wird dagegen eine Base benützt, so werden vorteilhaft Alkalibasen wie Natriumhydroxid oder Kaliumhydroxid eingesetzt. Als Lösungsmittel kann praktisch jedes, den Reaktionspartnern gegenüber inerte Lösemittel wie z. B. Alkanole, vorzugsweise Ethanol oder, bei Anwendung saurer Hydrolysebedingungen, Alkansäuren wie etwa Essigsäure benutzt werden. Im allgemeinen muß wenigstens 1 Äquivalent Wasser dem Reaktionsgemisch zugesetzt werden, meist wird jedoch ein größerer Überschuß verwendet oder Wasser allein dient als Lösemittel, was insbesondere bei alkalischen Verseifungen vorteilhaft ist. Die Reaktionstemperatur kann zwischen 20 und 150 °C liegen, vorteilhaft wird bei Siedetemperatur des verwendeten Lösemittels gearbeitet. Das Produkt fällt bei saurer Verseifung sofort oder nach Entfernung des Lösemittels kristallin in Form eines Säureadditionssalzes an. Es wird gegebenenfalls in üblicher Weise durch Umkristallisation aus einem geeigneten Lösemittel gereinigt.

Bei alkalischen Verseifungen dagegen wird im allgemeinen nach Neutralisation überschüssiger Base sofort das freie Benzylamin der allgemeinen Formel I erhalten.

Die Herstellung der Verbindungen der allgemeinen Formel IV geschieht in an sich bekannter Weise dadurch, daß man die Phenole der allgemeinen Formel II mit N-Hydroxymethylcarboxamiden III, vorzugsweise mit 2-Halogen-N-hydroxymethylacetamiden wie z. B. 2-Chlor-N-hydroxymethylacetamid nach Art der Tschemiac-Einhorn Reaktion unter Säurekatalyse umsetzt. Als effiziente Katalysatorsäuren eignen sich vor allem starke Mineralsäuren wie etwa Chlorwasserstoffsäure oder Schwefelsäure. Als Lösemittel dienen alle für derartige Reaktionen gebräuchlichen Lösemittel, als besonders geeignet erwiesen sich Alkansäuren wie Essigsäure oder Propionsäure, aber auch überschüssige Mineralsäure als Lösungsmittel wie z. B. reine konzentrierte Schwefelsäure kann vorteilhaft sein. Die Umsetzungen werden zwischen 0 und 100 °C vorgenommen, zur Vermeidung von Nebenprodukten vorteilhaft in einem Bereich von 0 bis 30 °C.

Unter Umständen kann es sinnvoll sein, das N-Hydroxymethylcarboxamid der Formel III in situ zu erzeugen, z. B. dadurch, daß man Phenole der Formel II mit einem Gemisch aus Carboxamid

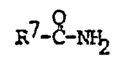

und einer Carbonylverbindung der Formel R—CHO, in der R die oben erwähnte Bedeutung hat, in der oben angegebenen Weise umsetzt, um Verbindungen der Formel IV zu erhalten.

Es zeigte sich insbesondere, daß derartige Amidomethylierungen bei genau eingehaltener Reaktionszeit, die je nach Verbindung und Reaktionstemperatur zwischen 10 Minuten und einigen Stunden liegen kann, auch dann noch gut durchführbar sind, wenn verschärfte Reaktionsbedingungen (wie etwa konz. Schwefelsäure als Lösemittel) angewandt werden, die sich unter Umständen als notwendig erweisen für einen raschen und vollständigen Umsatz. Zu erwartende Nebenreaktionen wie z. B. die Desalkylierung, insbesondere die De-tert.-butylierung können durch genau kontrollierte Reaktionsbedingungen auf ein Minimum reduziert werden.

Die Isolierung der Reaktionsprodukte geschieht am zweckmäßigsten dadurch, daß man ein Nichtlösemittel, wie etwa Wasser, dem Reaktionsgemisch beigefügt, sie fallen dann in der Regel sofort kristallin an und können nach Umkristallisation aus einem geeigneten Lösemittel oder in vielen Fällen ohne weitere Reinigung weiterverarbeitet werden.

Die für die Verfahrensweisen a und b benutzten Ausgangsverbindungen der allgemeinen Formel II können nach verschiedenen Verfahren hergestellt werden. Eine Methode besteht z. B. darin, daß man Thioether der allgemeinen Formel VIII oxidiert :

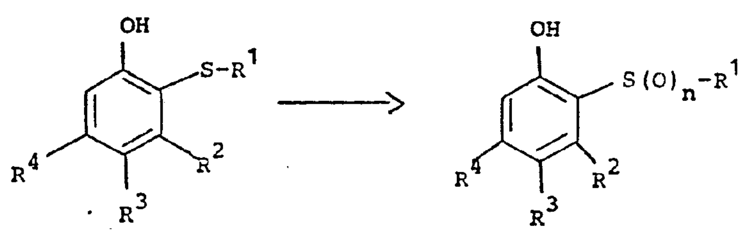

(VIII) (II)

wobei die Reste $R_1$ bis $R_4$ und n die oben erwähnten Bedeutungen haben. Derartige Oxidationen sind literaturbekannt. Weiterhin ist bekannt, daß man durch Wahl der Reaktionsbedingungen entweder Sulfoxide (n = 1) oder Sulfone (n = 2) erhalten kann.

Die Thioether der allgemeinen Formel VIII können in an sich bekannter Weise aus den Phenolen der Formel IX, in der $R^2$ bis $R^4$ obige Bedeutung hat, hergestellt werden ; z. B. durch Einwirken eines Sulfoxides $R^1$—SO—$R^1$ in Gegenwart von Perchlorsäure und Phosphoroxychlorid oder durch Reaktion mit einem Sulfenylchlorid $R^1$—S—Cl in an sich bekannter Weise, wobei $R^1$ jeweils die oben erwähnte Bedeutung hat.

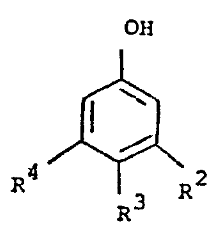

(IX)

Die Phenole IX können leicht durch Standardmethoden (vgl. Houben-Weyl, Methoden der org. Chem. — Phenole, Teil 2, S. 925 ff. G, Thieme Verlag, Stuttgart 1976) hergestellt werden.

Eine weitere Methode zur Herstellung von Phenolen der allgemeinen Formel II, in der $R^1$ bis $R^4$ die oben genannten Bedeutungen haben und n = 2 ist, besteht in der Etherspaltung von Anisolen der allgemeinen Formel X mit entsprechenden Bedeutungen von $R^1$ bis $R^4$ und n.

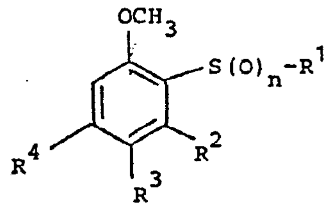

(X)

Sie wird in an sich bekannter Weise durch Einwirkung von Mineralsäuren wie Jodwasserstoffsäure oder von Lewissäuren wie Aluminiumchlorid oder Bortribromid in inerten Lösungsmittel, wie z. B. Methylenchlorid oder Chloroform, vorgenommen. Auch die gängige Spaltung durch Pyridiniumhydrochlorid bei Temperaturen oberhalb 180 °C kann erfolgreich durchgeführt werden.

Verbindungen der allgemeinen Formel X mit n = 2 werden durch eine Sequenz an sich bekannter Standardmethoden aus den Anisolen XI hergestellt:

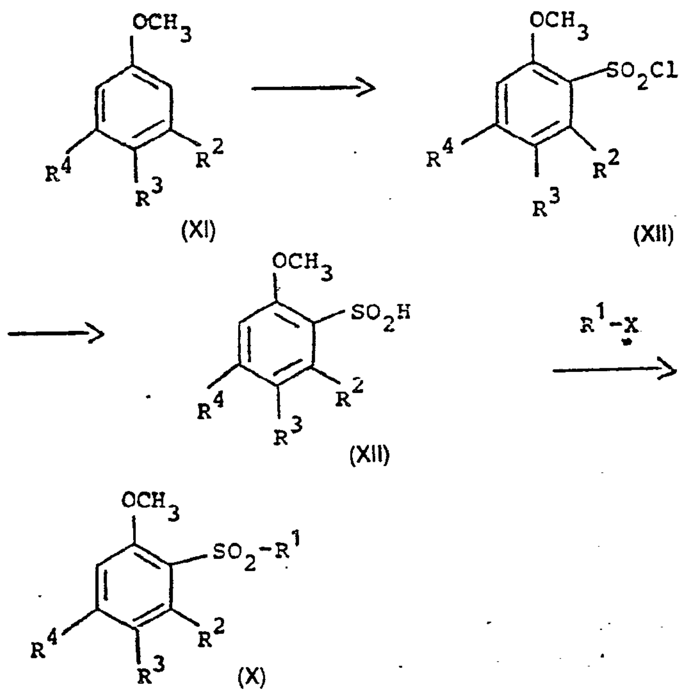


(XI) (XII) (XII) (X)

Durch Einwirkung von Chlorschwefelsäure auf Anisole XI erhält man in an sich bekannter Weise Sulfochloride XII. Vorteilhaft wird diese Reaktion in einem inerten Lösemittel wie Chloroform oder Methylenchlorid durchgeführt. Wenn $R^3$ in XI für höheres Alkyl steht, ist ein Temperaturbereich von 0-20 °C einzuhalten und ein an sich üblicher, größerer Überschuß von mehr als 3 Äquivalenten Chlorsulfonsäure zu vermeiden.

Die Reduktion zu den Sulfinsäuren XIII kann nach den verschiedensten Verfahren (vgl. Houben-Weyl, Methoden der org. Chemie, Band X, S. 563 ff., G. Thieme Verlag, Stuttgart, 1955) durchgeführt werden. Als einfache und effiziente Methode erwies sich die Reduktion mit Natriumsulfit in Gegenwart von Natriumhydroxid in wäßrig-acetonischen Lösungen.

Die Alkylierung von Sulfinsäuren zu Sulfonen ist eine an sich bekannte Reaktion, wobei vorzugsweise unter Basenkatalyse gearbeitet wird. Im Falle der Sulfinsäuren XIII erweisen sich Alkyljodide als besonders geeignete Alkylierungsmittel $R^1$—X, wobei $R^1$ die oben genannte Bedeutung hat und X für Halogen, vorzugsweise für Jod steht. Als Basen werden vorteilhaft organische Basen wie z. B. Triethylamin in acetonischer Lösung aber auch anorganische Basen wie LiOH, NaOH, KOH usw. in wäßriger, oder wäßrig-acetonischer Lösung eingesetzt.

Steht $R^1$ für einen α-Halogenalkylrest, so können eine Reihe Varianten dieser Alkylierungsreaktion vorteilhaft zur Anwendung kommen, wie z. B. die Umsetzung von Sulfinsäuren mit α-Dihalogencarbonsäuren in Gegenwart von $K_2CO_3$, wobei die intermediär gebildete β-Sulfonyl-α-halogencarbonsäure in an sich bekannter Weise $CO_2$ abspaltet unter Bildung des α-Halogenmethylsulfonylrestes.

Die Anisole XI werden durch Standardmethoden (Organikum, VEB Deutscher Verlag der Wissenschaften, Berlin 1971, S. 222) aus den Phenolen IX hergestellt.

Verbindungen der allgemeinen Formel I, in denen $R^5$ und $R^6$, wie eingangs definiert, beide Alkylreste sind oder cyclisch mitelnander verbunden sind, werden vorteilhaft nach Verfahren b hergestellt, indem man in an sich bekannter Weise die Phenole II nach Art der Mannich-Reaktion mit Aminen der allgemeinen Formel V umsetzt. Formaldehyd wird dabei bevorzugt in Form einer wäßrigen Lösung eingesetzt, es können jedoch auch alle anderen gängigen Varianten der Mannich-Reaktion zur Anwendung kommen, wie z. B. der Einsatz von Paraformaldehyd.

Auch das Lösemittel kann in weiten Grenzen variiert werden, besonders geeignete Lösemittel sind Alkanole wie etwa Methanol oder Ethanol. Man arbeitet in einem Temperaturbereich von 40-150 °C, bevorzugt in einem Bereich von 60 bis 100 °C. Die Reaktionszeit hängt entscheidend von der Temperatur ab, im allgemeinen sind die Umsetzungen nach wenigen Stunden beendet. Man isoliert die Produkte bevorzugt durch Abdampfen des Lösemittels und überschüssiger Reagentien, sie fallen meist als hochviskose Öle an, die durch Kristallisation aus einem geeigneten Lösemittel gereinigt werden oder sofort nach Verfahrensvariante c weiterverarbeitet werden.

Für die Herstellung von Benzylaminen I, besonders von solchen, bei denen $R^5$ und $R^6$ für Wasserstoff stehen oder die zusätzlich einen säureempfindlichen Rest wie etwa den Sulfinylalkylrest tragen, ist die Verfahrensvariante c besonders geeignet. Man geht dabei so vor, daß man die Verbindungen VI mit den Aminen H—N = Y mit oder ohne Basenkatalyse umsetzt. Die Art der Durchführung richtet sich dabei nach der Bedeutung von Y sowie nach der Natur der Fluchtgruppe Z in den Verbindungen VI.

Steht Y beispielsweise für den als Aminschutzgruppe besonders geeigneten Diazorest, so bedeutet dies, daß man aus den Verbindungen VI, bevorzugt aus solchen, bei denen Z für den Trialkylammoniumrest steht, beispielsweise für den Trimethylammoniumrest mit Halogenid, vorzugsweise Jodid als Gegenion, durch Einwirkung von Stickstoffwasserstoffsäure unter Basenkatalyse die Verbindungen XII erhält. Als Basen kommen organische Basen wie tertiäre Amine, z. B. Triethylamin oder quartäre Stickstoffbasen wie Tetraethylammoniumhydroxid in Frage aber auch anorganische Basen wie etwa Alkali- oder Erdalkalihydroxide können verwendet werden. Besonders vorteilhaft können auch unmittelbar die Salze der Stickstoffwasserstoffsäure eingesetzt werden, z. B. die Alkalisalze, wie etwa das Natriumazid.

Als Lösemittel dienen vorwiegend polar aprotische Solventien, wie Dimethylsulfoxid, Dimethylformamid oder Sulfolan, aber auch protische, polare Solventien wie Alkanole, z. B. Ethanol können mit oder ohne Wasserzusatz verwendet werden.

Die Reaktion wird dabei in einem Temperaturbereich von 40-150 °C durchgeführt, vorzugsweise bei Temperaturen zwischen 60 und 120 °C. Je nach Art des Lösemittels und Höhe der Temperatur ist die Reaktion meist nach wenigen Stunden beendet; in der Regel genügt einstündiges Erhitzen auf 100 °C. Durch Zusatz eines Nichtlösemittels wie etwa Wasser wird das Azid der allgemeinen Formel VII (Y ist = $N_2$) leicht in hinreichend reiner Form erhalten und sofort der Reduzierung zu den Aminen I unterworfen.

Es kommen dabei prinzipiell alle für derartige Umsetzungen bekannten Methoden, wie sie z. B. in H. Bayley, D.N. Standring, J.R. Knowles, Tetrahedron Letters *39*, 3633 (1978) zitiert sind, in Frage. Vorteilhaft wird dabei so vorgegangen, daß man das Azid einer katalytischen Hydrierung unterwirft. Als Lösemittel kommen praktisch alle bei Hydrierungen üblichen Lösemittel, beborzugt jedoch Alkanole wie etwa Methanol in Betracht. Auch der Hydrierkatalysator kann in weiten Grenzen variiert werden, vorteilhaft können z. B. alle Edelmetallkatalysatoren mit oder ohne Träger benutzt werden, z. B. Platindioxid, Rhodium, Palladium, Raney-Nickel, bevorzugt wird mit Palladium auf Tierkohle gearbeitet.

Die Hydrierung kann bei Temperaturen zwischen 0 und 100 °C und Drücken von 1 bis 100 atm durchgeführt werden, wobei vorzugsweise jedoch bei Normaldruck und Temperaturen zwischen 20 und 40 °C gearbeitet wird.

Das erhaltene Produkt bedarf im allgemeinen einer weiteren Reinigung, nachdem es durch Filtration und Abdampfen des Lösemittels isoliert wurde. Gut geeignet ist unter anderem die Chromatographie, vorzugsweise an Kieselgel und unter Anwendung polarer Elutionsmittel wie z. B. Aceton oder Methanol.

Verbindungen der allgemeinen Formel VI mit Z in der Bedeutung Trialkylammonium können leicht in Form ihrer Halogenide z. B. in Form ihrer Jodide erhalten werden, wenn man die nach Verfahrensvariante b erhaltenen Mannichbasen in an sich bekannter Weise quaternisiert mit Alkylierungsmitteln wie $R^8$—X, wobei $R^8$ für Alkyl mit 1 bis 4 C-Atomen, Benzyl oder Alkenyl mit 3 bis 6 C-Atomen steht und für $X^-$ bevorzugt das Anion einer Fluchtgruppe wie Halogen bedeutet, es kommen jedoch auch andere Fluchtgruppen wie etwa der Tosylrest, der Mesylrest oder der Methylsulfatrest in Frage. Bevorzugt sind dabei Methylierungsmittel wie Jodmethan. Bei der Umsetzung kann jedes den Reaktionspartnern gegenüber inerte Lösemittel Verwendung finden, z. B. Ketone, wie Aceton, Ester wie Ethylacetat oder chlorierte Kohlenwasserstoffe wie Methylenchlorid. Bevorzugt wird überschüssiges Alkylierungsmittel als Solvens verwendet. Im allgemeinen kann die Umsetzung im Temperaturbereich zwischen 0 und 150 °C durchgeführt werden, bei reaktiven Alkylierungsmitteln wie Jodmethan genügt meist Raumtemperatur, um die Reaktion in relativ kurzen Zeiten von wenigen Minuten bis Stunden ablaufen zu lassen.

Werden Verbindungen der Formel I nach Verfahrensvariante d) hergestellt, so erfolgt die Umsetzung der Carbonylverbindungen der Formel XIV mit Aminen der Formel $R_5$—$NH_2$ unter Bedingungen, die das Entstehen einer Schiffschen Base begünstigen (vgl. z.B. Chem. Reviews *63* [1963] 489-510). Im

einfachsten Fall geschieht dies durch Mischen der Ausgangskomponenten in eine geeigneten Lösemittel, beispielsweise in einem niederen Alkohol wie Methanol. Meist läuft die Reaktion schon bei Raumtemperatur ab, in wenigen Fällen sind höhere Reaktionstemperaturen notwendig. Gegebenenfalls müssen wasserentziehende Mittel zugesetzt werden, beispielsweise $TiCl_4$ oder Molekularsieb, oder das Reaktionswasser wird durch azeotrope Destillation entfernt. Für die anschließende Reduktion ist es im allgemeinen nicht notwendig, die Schiffsche Base zu isolieren, die anfallenden Lösungen dieser Verbindungen werden vielmehr sofort weiterreduziert, wobei alle für derartigen Umsetzungen bekannten Methoden in Frage kommen, z. B. katalytische Hydrierung oder Reduktion mit komplexen Hydriden wie etwa $NaBH_4$, $LiBH_4$, $LiAlH_4$ oder $NaBH_3CN$ (vgl. z. B. Houben-Weyl, Methoden der organischen Chemie, Bd. 11/1, Stuttgart 1957, Seite 341). Bevorzugt wird mit $NaBH_4$ in alkoholischer Lösung gearbeitet, wobei Raumtemperatur im allgemeinen ausreichend ist. Die Carbonylverbindungen der Formel XIV können nach Standardmethoden, beispielsweise durch Oxidation mit Dimethylsulfoxid oder mit Urotropin aus den Verbindungen der Formel I mit R, $R^5$, $R^6$ = H hergestellt werden.

Die nach den verschiedenen Verfahrensvarianten hergestellten Verbindungen der allgemeinen Formel I fallen entweder als freie Basen oder als Säureadditionssalze an.

Um aus einem Säureadditionssalz die freie Base zu erzeugen, ist es notwendig, das Salz mit wenigstens einem Äquivalent einer Base zu behandeln. Es kommen dabei sowohl organische als auch anorganische Basen in Frage, z. B. Triethylamin, Tetraethylammoniumhydroxid oder Piperidin bzw. Lithium-, Natrium-, Kaliumhydroxid, Natriumhydrogencarbonat oder Natriumcarbonat. Vorteilhaft wird bei dieser Reaktion das Saüreadditionssalz in gelöster Form eingesetzt, z. B. in Alkanolen wie Methanol oder Ethanol oder, was sich als besonders günstig erwies, in Form von wäßrigen Lösungen, bei denen der Zusatz von anorganischer Base wie etwa Natriumhydroxid ein Ausfällen von kristalliner freier Base I bewirkt.

Umgekehrt werden Säureadditionsprodukte mit einer gewünschten Säure HA dadurch hergestellt, daß man Lösungen der freien Base I mit wenigstens einem Äquivalent der Säure HA behandelt, bevorzugt sind entweder alkoholische Lösungen wie etwa eine methanolische Lösung oder aber wäßrige Lösungen. Das Säureadditionssalz kristallisiert entweder sofort oder nach Entfernen des Lösemittels, gegebenenfalls nach Umkristallisation aus einem geeigneten Lösemittel, in reiner Form aus.

Als Säuren HA für pharmazeutisch bevorzugte Säureadditionen kommen beispielsweise in Frage :

organische Säuren wie Weinsäure, Äpfelsäure. Milchsäure, Essigsäure, Citronensäure, Methansulfonsäure, Benzolsulfonsäure u. ä.,

anorganische Säuren wie Chlorwasserstoff-, Bromwasserstoff-, Jodwasserstoffsäure, Schwefelsäure, Salpetersäure, Phosphorsäure oder Amidosulfonsäure usw.

Erfindungsgemäß können außer den in den Ausführungsbeispielen beschriebenen Verbindungen auch die in der folgenden Tabelle zusammengestellten Verbindungen der allgemeinen Formel I erhalten werden :

(Siehe Tabelle Seite 9 ff.)

Tabelle

Am = Amyl    Bu = Butyl

| $R^1$ | $R^2$ | $R^3$ | $R^4$ | $R^5$ | $R^6$ | n | R |
|---|---|---|---|---|---|---|---|
| $C_2H_5$ | H | t-Am | H | H | H | 2 | H |
| $C_3H_7$ | H | t-Am | H | H | H | 2 | H |
| iso-$C_3H_7$ | H | t-Am | H | H | H | 2 | H |
| $CH_2$-CH=$CH_2$ | H | t-Am | H | H | H | 2 | H |
| $CH_3$-C=CH- | H | t-Am | H | H | H | 2 | H |
| $CH_2Cl$ | H | t-Am | H | H | H | 2 | H |
| $CH_2F$ | H | t-Bu | H | H | H | 2 | H |
| $CHF_2$ | H | t-Bu | H | H | H | 2 | H |
| $CF_3$ | H | t-Bu | H | H | H | 2 | H |
| $CH_2$-$CF_3$ | H | t-Bu | H | H | H | 2 | H |
| cyclo-$C_5H_9$ | H | t-Bu | H | H | H | 2 | H |
| cyclo-$C_6H_{11}$ | H | t-Bu | H | H | H | 2 | H |
| $C_2H_5$ | H | t-Bu | H | H | H | 1 | H |
| $C_3H_7$ | H | t-Bu | H | H | H | 1 | H |
| iso-$C_3H_7$ | H | t-Bu | H | H | H | 1 | H |
| $CH_2$-HC=$CH_2$ | H | t-Bu | H | H | H | 1 | H |
| $CH_2$-C=CH | H | t-Bu | H | H | H | 1 | H |
| $CH_2Cl$ | H | t-Bu | H | H | H | 1 | H |
| $CH_2F$ | H | t-Bu | H | H | H | 1 | H |
| $CHF_2$ | H | t-Bu | H | H | H | 1 | H |
| $CF_3$ | H | t-Bu | H | H | H | 1 | H |
| $CH_2CF_3$ | H | t-Bu | H | H | H | 1 | H |
| cyclo-$C_5H_9$ | H | t-Bu | H | H | H | 1 | H |
| $CH_2$=CH- | H | t-Bu | H | H | H | 2 | H |
| $CH_2$=CH- | H | t-Bu | H | H | H | 1 | H |
| $CH_3$-C=CH | H | t-Bu | H | H | H | 2 | H |

(Siehe Fortsetzung Seite 10 f.)

(Fortsetzung)

| $R^1$ | $R^2$ | $R^3$ | $R^4$ | $R^5$ | $R^6$ | n | R |
|---|---|---|---|---|---|---|---|
| $CH_3$ | H | t-Bu | H | $C_2H_5$ | $C_2H_5$ | 2 | H |
| $CH_3$ | H | t-Bu | H | $CH_3$ | $C_2H_5$ | 2 | H |
| $CH_3$ | H | t-Bu | H | H | $C_2H_5$ | 2 | H |
| $CH_3$ | H | t-Bu | H | $C_2H_3$ | $C_2H_5$ | 1 | H |
| $CH_3$ | H | t-Bu | H | $CH_3$ | $C_2H_5$ | 1 | H |
| $CH_3$ | H | t-Bu | H | H | $CH_3$ | 1 | H |
| $CH_3$ | H | t-Bu | H | H | $C_2H_5$ | 1 | H |
| $CH_3$ | H | t-Bu | H | $—C_4H_8—$ | | 1 | H |
| $CH_3$ | H | t-Bu | H | $—C_5H_{10}—$ | | 1 | H |
| $CH_3$ | H | sec.-Bu | H | H | H | 1 | H |
| $CH_3$ | H | $CCH_3(C_2H_5)_2$ | H | H | H | 1 | H |
| $CH_3$ | H | n-Bu | H | H | H | 1 | H |
| $CH_3$ | H | n-Bu | H | H | H | 2 | H |
| $CH_3$ | H | cyclo-$C_5H_9$ | H | H | H | 1 | H |
| $CH_3$ | H | cyclo-$C_6H_{11}$ | H | H | H | 1 | H |
| $CH_3$ | H | cyclo-$C_6H_{11}$ | H | H | H | 2 | H |
| $CH_3$ | H | cyclo-$C_7H_{13}$ | H | H | H | 1 | H |
| $CH_3$ | H | cyclo-$C_7H_{13}$ | H | H | H | 2 | H |
| $CH_3$ | H | t-Bu | H | $-(CH_2)_2-NH-(CH_2)_2-$ | | 2 | H |
| $CH_3$ | H | t-Bu | H | $-(CH_2)_2-NH-(CH_2)_2-$ | | 1 | H |
| $CH_3$ | H | t-Bu | H | H | $CH_3$ | 1 | H |

(Siehe Fortsetzung Seite 11 f.)

(Fortsetzung)

| $R^1$ | $R^2$ | $R^3$ | $R^4$ | $R^5$ | $R^6$ | n | R |
|---|---|---|---|---|---|---|---|
| $CH_3$ | H | iso-$C_3H_7$ | H | H | H | 1 | H |
| $CH_3$ | Cl | Cl | Cl | H | H | 1 | H |
| $CH_3$ | $CH_3$ | Cl | $CH_3$ | H | H | 1 | H |
| $CH_3$ | $CH_3$ | Br | $CH_3$ | H | H | 2 | H |
| $CH_3$ | $CH_3$ | Br | $CH_3$ | H | H | 1 | H |
| $CH_3$ | $C_2H_5$ | Cl | $C_2H_5$ | H | H | 1 | H |
| $CH_3$ | $OCH_3$ | Cl | $OCH_3$ | H | H | 1 | H |
| $CH_3$ | $OCH_3$ | t-Bu | $OCH_3$ | H | H | 2 | H |
| $CH_3$ | $OC_2H_5$ | $CH_3$ | $OC_2H_5$ | H | H | 2 | H |
| $CH_3$ | $OC_2H_5$ | $CH_3$ | $OC_2H_5$ | H | H | 1 | H |
| $CH_3$ | $CH_3$ | $CH_3$ | $CH_3$ | H | H | 1 | H |
| $CH_3$ | $CH_3$ | $CH_3$ | $CH_3$ | H | H | 2 | H |
| $CH_3$ | $CH_3$ | $CH_3$ | H | H | H | 2 | H |
| $CH_3$ | H | $CH_3$ | $CH_3$ | H | H | 2 | H |
| $CH_3$ | H | $CH_3$ | $CH_3$ | H | H | 1 | H |
| $CH_3$ | $CH_3$ | $CH_3$ | H | H | H | 1 | H |
| $CH_3$ | $-C_4H_8-$ | | H | H | H | 1 | H |
| $CH_3$ | H | $-C_4H_8-$ | | H | H | 1 | H |
| $CH_3$ | $-C_4H_8-$ | | H | H | H | 2 | H |
| $CH_3$ | $-C_3H_6-$ | | H | H | H | 2 | H |
| $CH_3$ | $-C_3H_6-$ | | H | H | H | 1 | H |
| $CH_3$ | H | $-C_3H_6-$ | | H | H | 1 | H |
| $CH_3$ | H | t-Am | H | H | H | 2 | $CH_3$ |
| $CH_3$ | H | iso-$C_3H_7$ | H | H | H | 2 | $CH_3$ |
| $CH_3$ | H | t-Bu | H | H | H | 1 | $CH_3$ |
| $CH_3$ | H | t-Bu | H | H | H | 2 | $C_2H_5$ |
| $CH_3$ | H | t-Bu | H | H | H | 1 | $C_2H_5$ |

(Siehe Fortsetzung Seite 12 f.)

(Fortsetzung)

| $R^1$ | $R^2$ | $R^3$ | $R^4$ | $R^5$ | $R^6$ | n | R |
|---|---|---|---|---|---|---|---|
| $CH_2-Br$ | H | t-Bu | H | H | H | 1 | H |
| $CH_2-Br$ | H | t-Bu | H | H | H | 1 | H |
| $CH_3$ | F | Cl | H | H | H | 2 | H |
| $CH_3$ | Cl | Cl | H | H | H | 2 | H |
| $CH_3$ | $CH_3$ | Cl | H | H | H | 2 | H |
| $CH_3$ | F | Cl | H | H | H | 1 | H |
| $CH_3$ | Cl | Cl | H | H | H | 1 | H |
| $CH_3$ | $CH_3$ | Cl | H | H | H | 1 | H |
| $CH_3$ | $OCH_3$ | Cl | H | H | H | 2 | H |
| $CH_3$ | $OCH_3$ | Cl | H | H | H | 1 | H |
| $CH_3$ | H | Cl | F | H | H | 2 | H |
| $CH_3$ | H | Cl | Cl | H | H | 2 | H |
| $CH_3$ | H | Cl | $CH_3$ | H | H | 2 | H |
| $CH_3$ | H | Cl | F | H | H | 1 | H |
| $CH_3$ | H | Cl | Cl | H | H | 1 | H |
| $CH_3$ | H | Cl | $CH_3$ | H | H | 1 | H |
| $CH_3$ | H | Cl | $OCH_3$ | H | H | 1 | H |
| $CH=CH_2$ | H | t-Bu | H | H | H | 1 | H |
| $CH=CH_2$ | H | t-Bu | H | H | H | 2 | H |
| Cyclo-$C_4H_7$ | H | t-Bu | H | H | H | 2 | H |
| Cyclo-$C_3H_5$ | H | t-Bu | H | H | H | 1 | H |

Die erfindungsgemäßen Verbindungen der Formel I sowie deren pharmazeutisch verträglichen Salze sind Diuretika, Salidiuretika und Antihypertensiva, die als Pharmazeutika in der Human- und Veterinärmedizin eingesetzt werden können. Sie werden in Dosierungen von 6-4 000 µg/kg.Tag, vorzugsweise 60-1 300 µg/kg.Tag, insbesondere 60-700 µg/kg.Tag in Kapseln, Dragees, Tabletten oder Lösungen mit verschiedenen Zusätzen enteral z. B. oral mit Sonde oder dgl. oder parenteral (Injektion in das Gefäßsystem, z. B. intravenös oder auch Injektion in die Muskulatur oder unter die Haut und dgl.) verabfolgt. Sie eignen sich sowohl zur Behandlung der Hypertonie als auch zur Behandlung von Ödemkrankheiten wie kardial, renal oder hepatisch bedingten Ödemen und anderen auf Störung des Wasser- und Elektrolyt-Haushalts zurückzuführenden Erscheinungen.

Die Verbindungen können allein oder in Kombination mit anderen salidiuretisch wirksamen Substanzen auch anderer Wirkungsart angewendet werden. Insbesondere sind zu nennen :

Spironolacton, Triamteren, Amilorid und andere $K^+$-retinierende Verbindungen oder langwirksame Salidiuretika vom Typ des Chlorthalidons. Aber auch andere, rein blutdrucksenkende Verbindungen kommen als mögliche Kombinationspartner in Frage, z. B. Hydralazin, Clonidin, Reserpin und insbesondere auch beta-blockierende Substanzen wie etwa Metoprolol oder Penbutolol.

Beispiel 1

2-Aminomethyl-4-(1,1-dimethylethyl)-6-methylsulfonylphenolhydrochlorid

a) 3-(1,1-Dimethylethyl)-6-methoxybenzolsulfonsäurechlorid

12,3 g (0,075 Mol) 4-(1,1-Dimethylethyl) anisol in 30 ml Methylenchlorid tropft man unter Eiskühlung zu 16,5 ml Chlorsulfonsäure gelöst in 20 ml Methylenchlorid. Man rührt das Gemisch 40 Minuten und gießt auf Eiswasser. Die organische Phase wird abgetrennt, mit Wasser gewaschen, mit $MgSO_4$ getrocknet und eingeengt. Die Umkristallisation erfolgt aus Toluol-Petrolether.

Kristalle vom Schmp. : 75-77 °C

b) 3-(1,1-Dimethylethyl)-6-methoxybenzolsulfinsäure

10,7 g (0,04 Mol) 3-(1,1-Dimethylethyl-6-methoxybenzolsulfonsäurechlorid werden in eine Lösung von 15 g $Na_2SO_3$ und 4 g NaOH in 100 ml Wasser eingetragen. Nach Zusatz von wenig Aceton wird die Mischung 30 Minuten auf dem Dampfbad erhitzt, filtriert und mit konz. Salzsäure auf pH 2-3 gebracht.

Farblose Kristalle vom Schmp. : 105-107 °C

c) 4-(1,1-Dimethylethyl)-2-methylsulfonylanisol

17,4 g (0,076 Mol) 3-(1,1-Dimethylethyl)-6-methoxybenzolsulfinsäure werden in 120 ml Aceton suspendiert, mit 13,3 ml (0,095 Mol) Triethylamin sowie mit 8,5 ml (0,12 Mol) Jodmethan versetzt. Die Mischung wird 2 Stunden bei Raumtemperatur gerührt und anschließend auf Eiswasser gegossen. Der ausgefallene Niederschlag wird abgesaugt und aus n-Butanol umkristallisiert.

Weiße Kristalle vom Schmp. : 111-112 °C

d) 4-(1,1-Dimethylethyl)-2-methylsulfonylphenol

23,1 g (0,095 Mol) 4-(1,1-Dimethylethyl)-2-methylsulfonylanisol werden mit 60 g Pyridiniumhydrochlorid vermischt und 2 Stunden auf 210-220 °C erhitzt. Die erkaltete Masse wird in Wasser suspendiert, der sich abscheidende Feststoff abgesaugt und mit Petrolether ausgekocht. Man engt bis zur beginnenden Kristallisation ein.

Weiße Nadeln vom Schmp. : 103-104 °C

e) 2-Chlor-N-[5-(1,1-dimethylethyl)-2-hydroxy-3-methylsulfonylbenzyl] acetamid

13,5 g (0,06 Mol) 4-(1,1-Dimethylethyl)-2-methylsulfonylphenol werden in 100 ml konz. Schwefelsäure gelöst. Man addiert 6,63 g (0,054 Mol) 2-Chlor-N-hydroxymethylacetamid und rührt 10 Minuten bei Raumtemperatur. Man gießt auf Eiswasser und kristallisiert das abgesaugte Rohprodukt aus Toluol um.

Farblose Kristalle vom Schmp. : 134-135 °C

f) 2-Aminomethyl-4-(1,1-dimethylethyl)-6-methylsulfonylphenolhydrochlorid

6,3 g (0,028 Mol) 2-Chlor-N-[5-(1,1-dimethylethyl)-2-hydroxy-3-methylsulfonylbenzyl] acetamid werden in einer Mischung aus 20 ml konz. Salzsäure und 40 ml Ethanol 16 Stunden unter Rückfluß gekocht. Das ausgefallene Produkt wird abgesaugt und aus Ethanol umkristallisiert. Farblose Nadeln vom Schmp. : 242-243 °C (Zers.)

Beispiel 2

2-Aminomethyl-4-isopropyl-6-methylsulfonylphenolhydrochlorid

a) 3-Isopropyl-6-methoxybenzolsulfonsäurechlorid

Aus 15 g (0,1 Mol) 4-Isopropylanisol und 20 ml Chloroschwefelsäure erhält man analog Beispiel 1a) das 3-Isopropyl-6-methoxybenzolsulfonsäurechlorid vom Schmp. : 60-61 °C.

b) 3-Isopropyl-6-methoxybenzolsulfinsäure

Aus 21,6 g (0,09 Mol) 3-Isopropyl-6-methoxybenzolsulfonsäurechlorid 32,6 g $Na_2SO_3$ und 100 ml 2n Natronlauge erhält man analog Beispiel 1b) die 3-Isopropyl-6-methoxybenzolsulfinsäure vom Schmp. 98-99 °C.

c) 4-Isopropyl-2-methylsulfonylanisol

21,4 g (0,1 Mol) 3-Isopropyl-6-methoxybenzolsulfinsäure, 17,32 ml (0,125 Mol) Triethylamin und 10,6 ml (0,17 Mol) Jodmethan ergeben analog Beispiel 1c) das 4-Isopropyl-2-methyl-sulfonylanisol vom Schmp. 50-52 °C.

d) 4-Isopropyl-2-methylsulfonylphenol

10,0 g (0,044 Mol) 4-Isopropyl-2-methylsulfonylanisol werden mit 36 g Pyridiniumhydrochlorid analog Beispiel 1.d) zum 4-Isopropyl-2-methylsulfonylphenol gespalten. Schmp. : 74-76 °C

e) 2-Chlor-N-(2-hydroxy-5-isopropyl-3-methylsulfonyl-benzyl) acetamid

6,8 g (0,032 Mol) 4-Isopropyl-2-methylsulfonylphenol werden in einer Mischung aus 40 ml Eisessig und 40 ml konz. Schwefelsäure gelöst und mit 4,73 g (0,038 Mol) 2-Chlor-N-hydroxymethylacetamid versetzt. Man rührt 15 Minuten bei Raumtemperatur, gießt die Mischung auf Eiswasser und saugt ab. Das als weißes Pulver anfallende 2-Chlor-N-(2-hydroxy-5-isopropyl-3-methylsulfonylbenzyl) acetamid vom Schmp. : 97-98 °C ist noch geringfügig mit Ausgangsmaterial verunreinigt. Es wird ohne weitere Reinigung in der nächsten Reaktion eingesetzt.

f) 2-Aminomethyl-4-isopropyl-6-methylsulfonylphenolhydrochlorid

Das bei der Reaktion 3e) angefallene 2-Chlor-N-(2-hydroxy-5-isopropyl-3-methylsulfonylbenzyl) acetamid wird analog Beispiel 1f) mit konz. Salzsäure in Ethanol behandelt. Nach Umkristallisation aus Ethanol erhält man das 2-Aminomethyl-4-isopropyl-6-methylsulfonylphenolhydrochlorid vom Schmp. : 246-247 °C (Zers).

Beispiel 3

2-Aminomethyl-4-(1,1-dimethylethyl)-6-isopropylsulfonylphenolhydrochlorid

a) 4-(1,1-Dimethylethyl)-2-isopropylsulfonylanisol

22,7 g (0,1 Mol) 3-(1,1-Dimethylethyl)-6-methoxybenzolsulfinsäure werden in 160 ml Aceton suspendiert und mit 19,35 ml (0,125 Mol) Triethylamin sowie mit 16,9 ml Isopropyljodid versetzt. Man rührt 1 Stunde bei Raumtemperatur und gießt anschließend auf Eiswasser. Man extrahiert dreimal mit Ethylacetat, engt die organische Phase stark ein und chromatographiert den Rückstand an Kieselgel, wobei mit Toluol/Ethylacetat eluiert wird.
Weiße Kristalle vom Schmp. : 75-76 °C

b) 4-(1,1-Dimethylethyl)-2-isopropylsulfonylphenol

4,6 g (0,017 Mol) 4-(1,1-Dimethylethyl)-2-isopropylsulfonylanisol werden in 50 ml Methylenchlorid gelöst und mit 2,5 ml Bortribromid (0,027 Mol) versetzt. Man rührt 1 Stunde bei Raumtemperatur, zerstört überschüssiges $BBr_3$ mit Methanol und zieht das Lösungsmittel im Vakuum ab. Der Rückstand wird mit Wasser verrieben und abgesaugt.
Weiße Kristalle vom Schmp. 54-55 °C

c) 2-Chlor-N-[5-(1,1-dimethylethyl)-2-hydroxy-3-isopropylsulfonylbenzyl] acetamid

4,1 g (0,016 Mol) 4-(1,1-Dimethylethyl)-2-isopropylsulfonylphenol werden in 36 ml konz. Schwefelsäure gelöst und mit 3,65 g (0,03 Mol) 2-Chlor-N-hydroxy-methylacetamid versetzt. Nach 10 Min. Rühren bei Raumtemperatur wird mit Eiswasser versetzt, der ausgeschiedene Kristallbrei abgesaugt und mit Methanol/Ether umkristallisiert.

Kristalle vom Schm. : 94-95 °C

d) 2-Aminomethyl-4-(1,1-dimethylethyl)-6-isopropylsulfonylphenolhydrochlorid

4,0 g (0,011 Mol) 2-Chlor-N-[5-(1,1-dimethylethyl)-2-hydroxy-3-isopropylsulfonylbenzyl] acetamid werden in 30 ml Ethanol und 10 ml konz. Salzsäure 8 Stunden am Rückfluß gekocht. Man engt ein, löst den Rückstand in 2n Salzsäure, schüttelt mehrfach mit Ethylacetat aus und zieht das Lösungsmittel in Vakuum ab. Der Rückstand wird mit Ether kristallisiert.

Weiße Kristalle vom Schmp. : 209-212 °C (Zers.)

Beispiel 4

2-Aminomethyl-4-ethyl-6-methylsulfonylphenolhydrochlorid

a) 3-Ethyl-6-methoxybenzolsulfonsäurechlorid

Aus 91 g (0,67 Mol) 4-Ethylanisol und 148 ml (1,5 Mol) Chloroschwefelsäure erhält man analog Beispiel 1.a) das 3-Ethyl-6-methoxybenzolsulfonsäurechlorid vom Schmp. : 61-62 °C.

b) 3-Ethyl-6-methoxybenzolsulfinsäure

68,5 g (0,29 Mol) 3-Ethyl-6-methoxybenzolsulfonsäurechlorid werden mit 108 g Natriumsulfit und 360 ml 2n Natronlauge analog Beispiel 1b) zur 3-Ethyl-6-methoxybenzolsulfinsäure reduziert.

Schmp. : 57-58 °C

c) 4-Ethyl-2-methylsulfonylanisol

2,0 g (0,01 Mol) 3-Ethyl-6-methoxybenzolsulfinsäure werden mit 1,73 ml (0,012 5 Mol) Triethylamin und 1,06 ml Methyljodid zum 4-Ethyl-2-methylsulfonylanisol umgesetzt. Das Rohprodukt wird durch Säulenchromatographie an Kieselgel mit dem Elutionsmittel Toluol/Essigester 4 : 1 gereinigt. Die Substanz fällt als hellgelbes Öl an.

d) 4-Ethyl-2-methylsulfonylphenol

6,0 g (0,028 Mol) 4-Ethyl-2-methylsulfonylanisol werden mit 18 g Pyridiniumhydrochlorid analog Beispiel 1d) zum 4-Ethyl-2-methylsulfonylphenol gespalten.

Schmp. : 101-102 °C

e) 2-Chlor-N-(2-hydroxy-5-ethyl-3-methylsulfonylbenzyl) acetamid

2,5 g (0,012 Mol) 4-Ethyl-2-methylsulfonylphenol werden in 30 ml konz. Schwefelsäure gelöst und mit 1,77 g (0,014 Mol) 2-Chlor-N-hydroxymethylacetamid versetzt. Man rührt 10 Minuten bei Raumtemperatur, gießt auf Eiswasser und extrahiert mit Ether. Man engt die mit Magnesiumsulfat getrockneten Etherauszüge ein, bis das 2-Chlor-N-(2-hydroxy-5-ethyl-3-methylsulfonylbenzyl) acetamid auskristallisiert. Farblose Kristalle vom Schmp. : 78-79 °C

f) 2-Aminomethyl-4-ethyl-6-methylsulfonylphenolhydrochlorid

2,4 g (0,008 Mol) 2-Chlor-N-(2-hydroxy-5-ethyl-3-methylsulfonylbenzyl) acetamid werden analog Beispiel 1.f) zum 6-Aminomethyl-4-ethyl-2-methylsulfonylphenolhydrochlorid verseift. Umkristallisation aus Methanol/Ether liefert farblose Kristalle von Schmp. : 210-211 °C (Zers.).

Beispiel 5

2-Aminomethyl-4-(1,1-dimethylethyl)-6-ethylsulfonylphenolhydrochlorid

a) 2-Ethylsulfonyl-4-(1,1-dimethylethyl) anisol

15,3 g (0,067 Mol) 3-(1,1-Dimethylethyl)-6-methoxybenzolsulfinsäure werden in 100 ml Aceton suspendiert, mit 12 ml Triethylamin und 8,6 ml (0,11 Mol) Ethylbromid versetzt und 1 Stunde bei Raumtemperatur gerührt. Man arbeitet analog Beispiel 1c) auf.

Schmp. : 104-105 °C

b) 2-Ethylsulfonyl-4-(1,1-dimethylethyl) phenol

6,0 g (0,038 Mol) 2-Ethylsulfonyl-4-(1,1-dimethylethyl)-anisol werden mit 18 g Pyridiniumhydrochlorid analog Beispiel 1.d) zum 2-Ethylsulfonyl-4-(1,1-dimethylethyl)-phenol umgesetzt.
Schmp.: 74-75 °C

c) 2-Chlor-N-[5-(1,1-dimethylethyl)-3-ethylsulfonyl-2-hydroxybenzyl] acetamid

5,7 g (0,026 Mol) 2-Ethylsulfonyl-4-(1,1-dimethylethyl)-phenol und 3,92 g (0,032 Mol) N-Hydroxymethylchloracetamid werden analog Beispiel 1e) zum 2-Chlor-N-[5-(1,1-dimethylethyl)-3-ethylsulfonyl-2-hydroxybenzyl] acetamid umgesetzt.
Weiße Kristalle vom Schmp.: 116-117 °C

d) 2-Aminomethyl-4-(1,1-dimethylethyl)-6-ethylsulfonylphenolhydrochlorid

2,4 g 2-Chlor-N-[5-(1,1-dimethylethyl)-3-ethylsulfonyl-2-hydroxybenzyl] acetamid werden in 25 ml EtOH und 2,5 ml konz. Salzsäure 8 Stunden unter Rückfluß gekocht. Aufarbeiten wie unter Beispiel 3d) liefert Kristalle vom Schmp.: 203-204 °C (Zers.)

Beispiel 6

2-Aminomethyl-6-chlormethylsulfonyl-4-(1,1-dimethylethyl)-phenolhydrochlorid

a) 2-Chlormethylsulfonyl-4-(1,1-dimethylethyl)anisol

22,8 g (0,1 Mol) 5-(1,1-Dimethylethyl)-2-methoxybenzolsulfinsäure werden mit 23 g $Na_2CO_3$ und 15 g (0,117 Mol) Dichloressigsäure in 150 ml $H_2O$ gelöst. Die Lösung wird bei einer Badtemperatur von 160 °C langsam zur Trockne eingedampft. Man nimmt erneut mit $H_2O$ auf, neutralisiert mit wenig verdünnter Salzsäure und schüttelt mehrfach mit Ethylacetat aus. Nach Entfernen des Lösungsmittels fällt das Produkt kristallin an.
Schmp.: 114-116 °C

b) 2-Chlormethylsulfonyl-4-(1,1-dimethylethyl)phenol

13,4 g (0,049 Mol) 2-Chlormethylsulfonyl-4-(1,1-dimethylethyl)anisol werden analog Beispiel 3b) zum 2-Chlormethylsulfonyl-4-(1,1-dimethylethyl)phenol gespalten.
Schmp.: 94-95 °C

c) 2-Chlor-N-[3-chlormethylsulfonyl-5-(1,1-dimethylethyl)-2-hydroxybenzyl] acetamid

Diese Verbindung wird analog Beispiel 1e) hergestellt, das Rohprodukt aus Toluol/Petrolether umkristallisiert.
Schmp.: 139-140 °C

d) 2-Aminomethyl-6-chlormethylsulfonyl-4-(1,1-dimethylethyl)phenolhydrochlorid

Diese Verbindung wird analog Beispiel 1d) hergestellt. Umkristallisation aus Methanol/Ether liefert weiße Kristalle vom Schmp.: 218-219 °C (Zers.)

Beispiel 7

2-Aminomethyl-4-methyl-6-methylsulfonylphenolhydrochlorid

a) 2-Chlor-N-(2-hydroxy-5-methyl-3-methylsulfonylbenzyl)acetamid

7,4 g (0,04 Mol) 4-Methyl-2methylsulfonylphenol werden in 70 ml konz. Schwefelsäure gelöst. Man addiert 4,4 g (0,036 Mol) 2-Chlor-N-hydroxymethylacetamid und rührt 10 Minuten bei Raumtemperatur. Man gießt auf Eiswasser, saugt den Niederschlag ab und kristallisiert aus Methanol um. Weiße Kristalle vom Schmp.: 115-116 °C.

b) 2-Aminomethyl-4-methyl-6-methylsulfonylphenolhydrochlorid

7,4 g (0,026 Mol) 2-Chlor-N-(2-hydroxy-5-methyl-3-methylsulfonylbenzyl)acetamid werden in 25 ml Ethanol und 3 ml konz. Salzsäure gelöst und 10 Stunden am Rückfluß erhitzt. Das Produkt wird abgesaugt und aus Methanol/Ether umkristallisiert.

Weiße Kristalle vom Schmp. : 227-228 °C

c) Herstellung der Ausgangsverbindung

Das 4-Methyl-2-methylsulfonylphenol von Beispiel 7a) wird analog der Reaktionsfolge 1a)-d) aus 4-Methylanisol hergestellt. Es weist einen Schmelzpunkt von 84-85 °C auf.

Beispiel 8

2-Aminomethyl-4-(1,1-dimethylpropyl)-6-methylbenzolsulfonsäurechlorid

a) 3-(1,1-Dimethylpropyl)-6-methoxybenzolsulfonsäurechlorid

Mit 4-(1,1-Dimethylpropyl)anisol als Ausgangsmaterial wird analog Beispiel 1a) sulfochloriert. Weiße Kristalle vom Schmp. : 40-42 °C.

b) 3-(1,1-Dimethylpropyl)-6-methoxybenzolsulfinsäure

Diese Verbindung wird analog Beispiel 1b) aus 3-(1,1-Dimethylpropyl)-6-methoxybenzolsulfonsäurechlorid hergestellt. Weiße Kristalle vom Schmp. : 91-92 °C

c) 4-(1,1-Dimethylpropyl)-2-methylsulfonylanisol

Die Verbindung wird aus 3-(1,1-Dimethylpropyl)-6-methoxybenzolsulfinsäure und Jodmethan analog Beispiel 1c) hergestellt.
Kristalle vom Schmp. : 64-65 °C

d) 4-(1,1-Dimethylpropyl)-2-methylsulfonylphenol

Die Verbindung wird aus 4-(1,1-Dimethylpropyl)-2-methylsulfonylanisol durch Einwirkung von Bortribromid analog Beispiel 3b) erhalten und fällt als hellgelbes Öl an.

e) 2-Chlor-N-[5-(1,1-dimethylpropyl)-2-hydroxy-3-methyl-sulfonylbenzyl] acetamid

8,5 g (0.035 Mol) 4-(1,1-Dimethylproyl)-2-methylsulfonylphenol werden in 80 ml konz. Schwefelsäure gelöst, mit 8 g (0,065 Mol) 2-Chlor-N-hydroxymethylacetamid versetzt und 10 Minuten bei Raumtemperatur gerührt. Man gießt auf Eiswasser, saugt ab und kristallisiert aus Toluol/Petrolether um.
Weiße Kristalle vom Schmp. : 119-120 °C.

f) 2-Aminomethyl-4-(1,1-dimethylpropyl)-6-methylsulfonylphenolhydrochlorid

Die Verbindung wird analog Beispiel 1f) hergestellt, jedoch mit 2-Chlor-N-[5-(1,1-dimethylpropyl)-2-hydroxy-3-methylsulfonylbenzyl] acetamid als Ausgangsmaterial.
Weiße Nadeln vom Schmp. : 187-188 °C (Zers.)

Beispiel 9

2-Aminomethyl-4-chlor-3,5-dimethyl-6-methylsulfonylphenohydrochlorid

a) 4-Chlor-3,5-dimethyl-2-methylsuflonylanisol

Diese Verbindung wird analog der Reaktionsfolge 1a)-1c) hergestellt, jedoch mit 4-Chlor-3,5-dimethylanisol als Ausgangsmaterial. Das Endprodukt schmilzt bei 129-130 °C.

b) 4-Chlor-3,5-dimethyl-2-methylsulfonylphenol

16,5 g 4-Chlor-3,5-dimethyl-2-methylsulfonylanisol (0,066 Mol) werden mit 50 g Pyridiniumhydrochlorid bei 190-210 °C umgesetzt. Der Reaktionsbrei wird mit Wasser verrieben und der entstandene Niederschlag abgesaugt.
Farblose Kristalle vom Schmp. : 119-120 °C.

c) 2-Chlor-N-[5-chlor-4,6-dimethyl-2-hydroxy-3-methylsulfonylbenzyl] acetamid

14,0 g (0,059 Mol) 4-Chlor-3,5-dimethyl-2-methylsulfonylphenol werden in 150 ml konz. Schwefelsäure gelöst, mit 8,7 g (0,07 Mol) 2-Chlor-N-hydroxymethylacetamid versetzt und 10 Minuten bei Raumtemperatur gerührt. Man rührt die Lösung in Eiswasser ein und saugt ab.
Weiße Kristalle vom Schmp. : 204-205 °C.

d) 2-Aminomethyl-4-chlor-3,5-dimethyl-6-methylsulfonylphenolhydrochlorid

16,0 g (0,048 Mol) 2-Chlor-N-[5-chlor-4,6-dimethyl-2-hydroxy-3-methylsulfonylbenzyl] acetamid werden in 120 ml Ethanol und 40 ml konz. Salzsäure 8 Stunden am Rückfluß gekocht. Nach dem Abkühlen im Eisbad wird abgesaugt und aus Methanol/Ether umkristallisiert.
Farblose Nadeln vom Schmp. : 259-260 °C (Zers.)

Beispiel 10

2-Aminomethyl-4-(1,1-dimethylethyl)-6-propylsulfonylphenohydrochlorid

a) 4-(1,1-Dimethylethyl)-2-propylsulfonylanisol

22,8 g (0,1 Mol) 3-(1,1-Dimethylethyl)-6-methoxybenzolsulfinsäure werden mit n-Propyljodid in Gegenwart von Triethylamin analog Beispiel 1c) zum 4-(1,1-Dimethylethyl)-2-propylanisol umgesetzt.
Weiße Kristalle vom Schmp. : 65-66 °C.

b) 4-(1,1-Dimethylethyl)-2-propylsulfonylphenol

7 g (0,026 Mol) 4-(1,1-Dimethylethyl)-2-propylsulfonylanisol werden analog Beispiel 1d) zum Phenol gespalten. Die Substanz fällt als hellgelbes Öl an, das ohne weitere Reinigung in der nächsten Reaktion eingesetzt wird.

c) 2-Chlor-N-[5-(1,1-dimethylethyl)-2-hydroxy-3-propylsulfonylbenzyl] acetamid

5,1 g (0,02 Mol) 4-(1,1-Dimethylethyl)-2-propylsulfonylphenol werden in 45 mol konz. Schwefelsäure gelöst, mit 4,56 g (0,037 Mol) 2-Chlor-N-hydroxymethylacetamid versetzt und 10 Minuten bei Raumtemperatur gerührt. Man gießt auf Eiswasser, saugt ab und kristallisiert mit Ether.
Weiße Kristalle von Schmp. : 108-110 °C.

d) 2-Aminomethyl-4-(1,1-dimethylethyl)-6-propylsulfonylphenolhydrochlorid

2,35 g (0,007 Mol) 2-Chlor-N-[5-(1,1-dimethylethyl)-2-hydroxy-3-propylsulfonylbenzyl] acetamid werden in einer Mischung aus 20 ml Ethanol und 5 ml konz. Salzsäure 8 Stunden am Rückfluß gekocht. Das Lösungsmittelgemisch wird am Rotationsverdampfer abgezogen, der Rückstand aus Methanol/Ether umkristallisiert.
Weiße Nadeln vom Schmp. : 189-190 °C (Zers.)

Beispiel 11

2-N,N-Dimethylaminomethyl-4-(1,1-dimethylethyl)-6-methylsulfonylphenolhydrochlorid

4,56 g (0,02 Mol) 4-(1,1-Dimethylethyl)-2-methylsulfonylphenol werden in 30 ml Ethanol gelöst, mit 5,6 ml (0,04 Mol) 40 %iger wässriger Dimethylaminlösung und 4 ml (0,04 Mol) 35 %iger wässriger Formaldehydlösung versetzt. Man kocht 1 Stunde am Rückfluß, engt die Lösung am Rotationsverdampfer bis zur Trockne ein und nimmt den Rückstand in 2n Salzsäure auf. Man schüttelt mehrfach mit Ethylacetat aus und engt die wässrige Phase bis zur Trockne ein. Der Rückstand wird aus Isopropanol/Ether umkristallisiert.
Weiße Kristalle vom Schmp. : 218-220 °C.

Beispiel 12

Methyl-[3-aminomethyl-5-(1,1-dimethylethyl)-2-hydroxyphenyl] sulfoxid hydrochloriddihydrat

a) Methyl-[5-(1,1-dimethylethyl)-2-hydroxyphenyl] sulfoxid

52 g (0,27 Mol) 4-(1,1-Dimethylethyl)-2-hydroxythioanisol werden in 300 ml Eisessig gelöst. Unter Eiskühlung werden 30 ml 30 % $H_2O_2$ zugetropft. Man rührt 2 Stunden bei Raumtemperatur, geißt auf Eiswasser und saugt ab. Umkristallisation aus Toluol.
Weiße Kristalle vom Schmp. : 149-150 °C.

b) [5-(1,1-Dimethylethyl)-2-hydroxy-3-methylsulfoxybenzyl] trimethylammoniumjodid

11 g (0,052 Mol) Methyl-[5-(1,1-dimethylethyl)-2-hydroxyphenyl] sulfoxid werden mit 14 ml (0,1 Mol) 40 %iger wässriger Dimethylaminlösung und 10 ml 35 %iger wässriger Formaldehydlösung (0,1 Mol) in 100 ml Ethanol 1 Stunde am Rückfluß gekocht. Man entfernt das Lösungsmittel am Rotationsverdampfer, nimmt in 2n Salzsäure auf und extrahiert mit Ethylacetat. Die wässrige Phase wird erneut bis zur Trockne eingedampft, der Rückstand in Aceton aufgenommen und mit 30 ml Jodmethan versetzt. Man beläßt ca. 1 Stunde bei Raumtemperatur und engt ein bis zur beginnenden Kristallisation.
Weiße Kristalle vom Schmp. : 183-185 °C.

c) Methyl-[3-azidomethyl-5-(1,1-dimethylethyl)-2-hydroxyphenyl] sulfoxid

8,9 g (0,022 Mol) des unter 11b) erhaltenen Ammoniumjodids werden in 80 ml Dimethylformamid gelöst, mit 5 g (0,08 Mol) Natriumazid versetzt und 30 Minuten bei 100 °C gerührt. Anschließend gießt man auf Eiswasser und saugt ab. Das noch schwach feuchte, gelbliche Produkt wird ohne weitere Reinigung in 80 mol Methanol gelöst.

d) Methyl-[3-aminomethyl-5-(1,1-dimethylethyl)-2-hydroxyphenyl] sulfoxidhydrochloriddihydrat

Die unter 11c) erhaltene methanolische Lösung von Methyl-[3-azidomethyl-5-(1,1-dimethylethyl)-2-hydroxyphenyl]-sulfoxid wird mit einer Suspension von 1 g 10 % Palladium auf Kohle vereint und 2 Stunden bei Raumtemperatur und Normaldruck hydriert. Man filtriert, engt ein und chromatographiert den Rückstand an Kieselgel, wobei ein Gemisch aus Ethylacetat und Methanol (2 : 1) als Elutionsmittel dient. Es fällt zunächst das freie Amin an (Schmp. : 192-193 °C), das durch Umkristallisation aus 2n Salzsäure in die Titelverbindung übergeht.
Weiße Kristalle vom Schmp. : 84-86 °C.

Beispiel 13

4-Aminomethyl-5-hydroxy-6-methylsulfonylindanhydrochlorid

a) 6-Chlorsulfonyl-5-methoxyindan

50 g (0,34 Mol) 5-Methoxyindan werden analog Beispiel 1a) mit 75 ml Chlorsulfonsäure zum 6-Chlorsulfonyl-5-methoxyindan umgesetzt.
Weiße Kristalle vom Schmp. : 75-77 °C.

b) 5-Methoxy-6-sulfinoindan

Aus 6-Chlorsulfonyl-5-methoxyindan wird analog Beispiel 1b) die Sulfinsäure erhalten.
Weiße Kristalle vom Schmp. : 105-106 °C.

c) 5-Methoxy-6-methylsulfonylindan

Aus 5-Methoxy-6-sulfinoindan analog Beispiel 1c) erhält man 5-Methoxy-6-methylsulfonylindan vom Schmp. : 108-110 °C.

d) 5-Hydroxy-6-methylsulfonylindan

Die Verbindung wird aus 5-Methoxy-6-methylsulfonylindan durch Einwirken von Bortribromid analog Beispiel 3b) erhalten.
Weiße Kristalle vom Schmp. : 149-151 °C.

e) 4-(N-Chloracetylaminomethyl)-5-hydroxy-6-methylsulfonylindan

13,5 g (0,064 Mol) 5-Hydroxy-6-methylsulfonylindan werden in 100 ml Schwefelsäure gelöst und mit 6 g 2-Chlor-N-hydroxymethylacetamid (0,048 Mol) versetzt. Man rührt 10 Minuten bei Raumtemperatur und gießt die Mischung auf Eiswasser. Man saugt ab und kristallisiert aus Ethanol um.
Weiße Kristalle vom Schmp. : 152-153 °C.

f) 4-Aminomethyl-5-hydroxy-6-methylsulfonylindanhydrochlorid

Die Verbindung wird analog Beispiel 1f) aus 4-(N-Chloracetylaminomethyl)-5-hydroxy-6-methylsulfonylindan hergestellt.
Weiße Kristalle vom Schmp. : 244-248 °C (Zers.).

## Beispiel 14

2-Aminomethyl-4-brom-6-methylsulfonylphenolhydrochlorid

a) 2-Chlor-N-(2-hydroxy-5-brom-3-methylsulfonylbenzyl)-acetamid

8,2 g (0,035 Mol) 4-Brom-2-methylsulfonylphenol werden in 82,5 ml konz. Schwefelsäure gelöst und mit 4,86 g (0,039 Mol) 2-Chlor-N-hydroxymethylacetamid versetzt. Man rührt ca. 30 Minuten bei Raumtemperatur und gießt die Mischung auf Eiswasser. Der abgesaugte Niederschlag wird aus Ethanol umkristallisiert.
Schmp. : 124-125 °C.

b) 2-Aminomethyl-4-brom-6-methylsulfonylphenolhydrochlorid

Diese Verbindung wird analog Beispiel 1f) aus 2-Chlor-N-(2-hydroxy-5-brom-3-methylsulfonylbenzyl)-acetamid hergestellt.
Schmp. : 228-229 °C (Zers.).

c) Herstellung der Ausgangsverbindung

Das 4-Brom-2-methylsulfonylphenol von Beispiel 15a) wird analog der Reaktionsfolge 1a)-d) aus 4-Bromanisol hergestellt.
Es hat einen Schmelzpunkt von 63-65 °C.

## Beispiel 15

2-Aminomethyl-4-(1-methylpropyl)-6-methylsulfonylphenol-hydrochlorid

a) 2-Chlor-N-[2-hydroxy-5-(1-methylpropyl)-3-methylsulfonylbenzyl] acetamid

Die Verbindung wird analog Beispiel 1e) hergestellt, jedoch mit 4-(1-methylpropyl)-2-methylsulfonylphenol als Ausgangsmaterial. Das Rohprodukt wird aus Ether/Petrolether umkristallisiert.
Schmp. : 61-62 °C.

b) 2-Aminomethyl-4-(1-methylpropyl)-6-methylsulfonylphenolhydrochlorid

Die Herstellung erfolgt analog Beispiel 1d). Weiße Kristalle vom Schmp. : 236-238 °C.

c) Herstellung der Ausgangsverbindung

Das 4-(1-Methylpropyl)-2-methylsulfonylphenol wird analog der Reaktionsfolge 1a)-1d) aus 4-(1-Methylpropyl) anisol hergestellt.
Schmp. : 58-59 °C.

## Beispiel 16

2-Aminomethyl-3,4,5-trichlor-2-methylsulfonylphenolmethansulfonat

a) 2-Chlor-N-(2-Hydroxy-4,5,6-trichlor-3-methylsulfonylbenzyl) acetamid

Diese Verbindung wird analog Beispiel 1e) hergestellt, jedoch mit 3,4,5-Trichlorphenol als Ausgangsmaterial.
Schmp. : 224-225 °C.

b) 2-Aminomethyl-3,4,5-trichlor-2-methylsulfonylphenolmethansulfonat

Die Herstellung verläuft analog Beispiel 1d), jedoch unter Verwendung von 20 % wäßriger Methansulfonsäure anstelle von konz. Salzsäure.
Schmp. : 253-254 °C.

c) Herstellung der Ausgangsverbindung

Das unter 16a benötigte 3,4,5-Trichlor-2-methylsulfonylphenol wird analog der Reaktionsfolge 1a)-1d) hergestellt.
Schmp. : 146-147 °C.

Beispiel 17

2-(1-Pyrrolidinyl)methyl-4-(1,1-dimethylethyl)-6-methylsulfonylphenol-hydrochlorid

Diese Verbindung wird analog Beispiel 11 hergestellt. Anstelle einer Dimethylaminlösung verwendet man jedoch Pyrrolidin. Umkristallisation aus Isopropanol/Ether.

Weiße Kristalle vom Schmp. : 230-231 °C.

Beispiel 18

2-(3-Thiazolidinyl)methyl-4-(1,1-dimethylethyl)-6-methylsulfonylphenol-hydrochlorid

Herstellung analog Beispiel 11 unter Verwendung von Thiazolidin anstelle von Dimethylamin. Weiße Kristalle vom Schmp. : 207-208 °C.

Beispiel 19

2-(4-Thiomorpholinyl)methyl-4-(1,1-dimethylethyl)-6-methylsulfonylphenol-hydrochlorid

Analog Beispiel 11 unter Verwendung von Thiomorpholin anstelle von Dimethylamin. Weiße Kristalle vom Schmp. : 249-250 °C.

Beispiel 20

2-(4-Morpholinyl)methyl-4-(1,1-dimethylethyl)-6-methylsulfonylphenol-hydrochlorid

Analog Beispiel 11 unter Verwendung von Morpholin anstelle von Dimethylamin. Weiße Kristalle vom Schmp. : 246-247 °C.

Beispiel 21

2-(4-Piperidinyl)methyl-4-(1,1-dimethylethyl)-6-methylsulfonylphenol-hydrochlorid

Analog Beispiel 11 unter Verwendung von Piperidin anstelle von Dimethylamin. Weiße Kristalle vom Schmp. : 233-234 °C.

Beispiel 22

2-Hexamethyleniminomethyl-4-(1,1-dimethylethyl)-6-methylsulfonylpehnol-hydrochlorid

Analog Beispiel 11 unter Verwendung von Hexamethylenimin anstelle von Dimethylamin.

Schmp. : 174-175 °C.

Beispiel 23

2-Aminomethyl-4-(1-methylcyclohexyl)-6-methylsulfonylphenol-hydrochlorid

a) 2-Chlor-N-[2-hydroxy-5-(1-methylcyclohexyl)-3-methylsulfonylbenzyl] acetamid

Diese Verbindung wird analog 1e) hergestellt, jedoch mit 4-(1-Methylcyclohexyl)-2-methylsulfonylphenol als Ausgangsmaterial. Das Rohprodukt wird durch Verreiben mit Ether zur Kristallisation Gebracht.

Schmp. : 146-148 °C.

b) 2-Aminomethyl-4-(1-methylcyclohexyl)-6-methylsulfonylphenol-hydrochlorid

Analog Beispiel 1d)

Weiße Kristalle vom Schmp. : 226-227 °C.

c) Herstellung der Ausgangsverbindung

Das unter 23a) benötigte 4-(1-Methylcyclohexyl)-2-methylsulfonylphenol wird analog Beispiel 1a)-1d) hergestellt.

Schmp. : 67-68 °C.

Beispiel 24

2-Aminomethyl-4-(1,1-diethylethyl)-6-methylsulfonylphenol-hydrochlorid

a) 2-Chlor-N-[2-hydroxy-5-(1,1-diethylethyl)-3-methylsulfonylbenzyl] phenolhydrochlorid

Analog Beispiel 1e), jedoch unter Verwendung von 4-(1,1-diethylethyl)-2-methylsulfonylphenol-hydrochlorid als Ausgangsmaterial. Umkristallisation aus Ether.
Schmp.: 117-118 °C.

b) 2-Aminomethyl-4-(1,1-diethylethyl)-6-methylsulfonylphenol-hydrochlorid

Analog Beispiel 1f)
Weiße Kristalle vom Schmp.: 179-181 °C.

c) Herstellung der Ausgangsverbindung

Das unter 24a) benötigte 4-(1,1-diethylethyl)-2-methylsulfonylphenol wird analog Beispiel 1a)-1d) hergestellt.
Schmp.: 84-86 °C.

Beispiel 25

2-Aminomethyl-3,5-dimethoxy-4-chlor-6-methylsulfonylphenol-hydrochlorid

a) 2-Chlor-N-(2-hydroxy-4,6-dimethoxy-5-chlor-3-methylsulfonylbenzyl)acetamid

Analog Beispiel 1e), jedoch unter Verwendung von 3,5-Dimethoxy-4-chlor-2-methylsulfonylphenol als Ausgangsmaterial. Die Reaktionsmischung wird außerdem auf 60 °C für die Dauer von ca. 15 Minuten erwärmt. Das Rohprodukt wird durch Auskochen mit n-Butanol gereinigt.
Schmp.: 181-182 °C.

b) 2-Aminomethyl-3,5-dimethoxy-4-chlor-6-methylsulfonylphenol-hydrochlorid

Analog Beispiel 1f). Umkristallisation aus Methanol/Ether liefert Kristalle vom Schmp.: 159-160 °C.

c) Herstellung der Ausgangsverbindung

Das unter 25a) benötigte 3,5-Dimethoxy-4-Chlor-2-methylsulfonylphenol wird analog Beispiel 12a), jedoch unter Verwendung von 2-Hydroxy-4,6-dimethoxy-5-chlorthioanisol als Ausgangsmaterial und von Trifluoressigsäure anstelle von Essigsäure als Lösemittel, hergestellt.
Schmp.: 170-172 °C.

Beispiel 26

2-Aminomethyl-4-(1,1-dimethylethyl)-6-jodmethylsulfonylphenol-hydrochlorid

a) 2-Chlor-N-[2-hydroxy-5-(1,1-dimethylethyl)-3-jodmethylsulfonylbenzyl] acetamid

Analog Beispiel 1e), jedoch unter Verwendung von 4-(1,1-Dimethylethyl)-2-jodmethylsulfonylphenol. Das Rohprodukt wird aus Isopropanol umkristallisiert.
Schmp.: 118-119 °C.

b) 2-Aminomethyl-4-(1,1-dimethylethyl)-6-jodmethylsulfonylphenol-hydrochlorid

Analog Beispiel 1f). Weiße Kristalle vom Schmp.: 220-223 °C.

c) Herstellung der Ausgangsverbindung

4-(1,1-Dimethylethyl)-2-jodmethylsulfonylphenol wird analog Beispiel 1d) hergestellt aus 4-(1,1-Dimethylethyl)-2-jodmethylsulfonylanisol und schmilzt bei 82-84 °C. Das 4-(1,1-Dimethylethyl)-2-jodmethylsulfonylanisol wird in literaturbekannter Weise aus der unter 1f) beschriebenen Sulfinsäure und Methylenjodid hergestellt.
Schmp.: 98-102 °C.

Beispiel 27

2-Aminomethyl-4-(1,1-dimethylethyl)-6-(2-methylpropyl)-sulfonylphenol-hydrochlorid

a) 2-Chlor-N-[2-hydroxy-5-(1,1-dimethylethyl)-3-(2-methyl-propyl)sulfonylbenzyl] acetamid

Analog Beispiel 1e), jedoch unter Verwendung von 4-(1,1-Dimethylethyl)-2-(2-methylpropyl)-sulfonylphenol als Ausgangsmaterial.
Schmp. : 97-98 °C.

b) 2-Aminomethyl-4-(1,1-dimethylethyl)-6-(2-methylpropyl)sulfonylphenol-hydrochlorid

Analog Beispiel 1f). Schmp. : 216-217 °C.

c) Herstellung der Ausgangsverbindung

4-(1,1-Dimethylethyl)-2-(2-methylpropyl)-sulfonylphenol von 27a) wird analog Beispiel 1c) und 1d) aus 2-Methoxy-5-(1,1-dimethylethyl)-benzolsulfinsäure und 1-Jod-2-methylpropan anstelle von Jodmethan hergestellt.
Schmp. : 52-53 °C.

Beispiel 28

2-Aminomethyl-4-(1,1-dimethylethyl)-6-allylsulfonylphenol-hydrochlorid

a) 2-Chlor-N-[2-hydroxy-5-(1,1-dimethylethyl)-3-allyl-sulfonylbenzyl] acetamid

Analog Beispiel 1e), jedoch unter Verwendung von 4-(1,1-Dimethylethyl)-2-allylsulfonylphenol als Ausgangsmaterial.
Schmp. : 119-120 °C.

b) 2-Aminomethyl-4-(1,1-dimethylethyl)-6-allylsulfonylphenol-hydrochlorid

Analog Beispiel 1f). Schmp. : 242-245 °C.

c) Herstellung der Ausgangsverbindung

4-(1,1-Dimethylethyl)-2-allylsulfonylphenol von Beispiel 28a) wird analog Beispiel 1c) und 1d) hergestellt.
Schmp. : 63-64 °C.

Beispiel 29

2-Aminomethyl-4-(1,1-dimethylethyl)-6-cyclopropylsulfonylphenol-hydrochlorid

a) 2-Chlor-N-[2-hydroxy-5-(1,1-dimethylethyl)-3-cyclopropylsulfonylbenzyl] acetamid

Analog Beispiel 1e), jedoch unter Verwendung von 4-(1,1-Dimethylethyl)-2-cyclopropylsulfonylphenol als Ausgangsmaterial. Das Rohprodukt wird aus Ether umkristallisiert.
Schmp. : 96-97 °C.

b) 2-Aminomethyl-4-(1,1-dimethylethyl)-6-cyclopropylsulfonylphenol-hydrochlorid

Analog Beispiel 1f), Umkristallisation des Rohprodukts aus Isopropanol/Ether.
Schmp. : 159-160 °C.

c) Herstellung der Ausgangsverbindung

26,5 g der unter 1b) erhaltenen Sulfinsäure (0,115 Mol) 87,5 g (0,1175 Mol) 20 % wäßrige Tetraethylammoniumhydroxidlösung sowie 55,5 g (0,275 Mol) Dibrompropan werden in 100 ml Dichlorethan 4 h am Rückfluß erhitzt. Die organische Phase wird abgetrennt und mit Toluol/Essigester 4 : 1 an Kieselgel chromatographiert.

Man erhält das 4-(1,1-Dimethylethyl)-2-(3-brompropyl)-sulfonylanisol, welches mit 1,1 Äquivalenten Kalium-tert.-butylat in siedendem tert. Butanol zum 4-(1,1-Dimethylethyl)-2-cyclopropylanisol umgesetzt. Aus diesem erhält man analog Beispiel 1d) das in Beispiel 29a) benötigte 4-(1,1-Dimethylethyl)-2-cyclopropylsulfonylphenol vom Schmp. : 85-86 °C.

## Beispiel 30

1-Aminomethyl-3-methylsulfonyl-5,6,7,8-tetrahydro-2-naphthol-hydrochlorid

a) 1-Chloracetylaminomethyl-3-methylsulfonyl-5,6,7,8-tetrahydro-2-naphthol

Diese Verbindung wird analog Beispiel 1e) hergestellt. Das Rohprodukt wird aus Ethanol umkristallisiert.
Schmp. : 173-175 °C.

b) 1-Aminomethyl-3-methylsulfonyl-5,6,7,8-tetrahydro-2-naphthol-hydrochlorid

Analog Beispiel 1f). Das Rohprodukt wird durch Verkochen mit Aceton gereinigt.
Schmp. : 238-242 °C.

c) Herstellung der Ausgangsverbindung

Das in Beispiel 30a) benötigte Ausgangsmaterial 3-Methylsulfonyl-5,6,7,8-tetrahydro-2-naphthol wird analog der Reaktionsfolge 1a) bis 1d) hergestellt aus 2-Methoxy-5,6,7,8-tetrahydronaphthalin und schmilzt bei 132-135 °C.

## Beispiel 31

2-(1-Aminoethyl)-4-(1,1-dimethylethyl)-6-methylsulfonylphenol-hydrochlorid

a) 2-(1-Chloracetylaminoethyl)-4-(1,1-dimethylethyl)-6-methylsulfonylphenol

5,7 g (0,025 Mol) 4-(1,1-Dimethylethyl)-2-methylsulfonylphenol, 2,25 g (0,025 Mol) Chloracetamid sowie 1,1 g (0,025 Mol) Acetaldehyd werden in 30 mol konz. Schwefelsäure gelöst und 1 h bei 0 °C gerührt. Man gießt auf Eiswasser, extrahiert mit Ethylacetat, trocknet mit $MgSO_4$ und engt ein. Durch Säulenchromatographie an Kieselgel erhält man mit dem Elutionsmittel Petrolether/Ethylacetat 4 : 1 zunächst 2,3 g Ausgangsmaterial sowie anschließend das gewünschte Produkt.
Schmp. : 137-139 °C.

b) 2-(1-Aminomethyl)-4-(1,1-dimethylethyl)-6-methylsulfonylphenol-hydrochlorid

Analog Beispiel 1f). Das Rohprodukt wird aus Isopropanol umkristallisiert.
Schmp. : 205-206 °C.

## Beispiel 32

2-Aminomethyl-4-(1,1-dimethylpropyl)-6-butylsulfonylphenol-hydrochlorid

Die Herstellung erfolgt analog der Reaktionsfolge 1a) bis 1f), jedoch wird auf Stufe 3 (analog 1c) Butyljodid anstelle von Methyljodid und auf Stufe 1 (analog 1a) 4-(1-Methylpropyl) phenol anstelle von 4-(1,1-Dimethylethyl)-phenol eingesetzt. Alle zwischenprodukte fielen als Öle an. Das Endprodukt schmilzt bei 148-151 °C.

## Beispiel 33

Methyl-[3-dimethylaminomethyl-5-(1,1-dimethylethyl)-2-hydroxyphenyl] sulfoxid

4,24 g (0,02 Mol) Methyl-[5-(1,1-Dimethylethyl)-2-hydroxypehnyl] sulfoxid werden in 30 ml Ethanol gelöst, mit 2,2 g Dimethylaminlösung (40 % in $H_2O$) sowie mit 2 ml (0,02 Mol) Formaldehydlösung (35 % in $H_2O$) versetzt. Man koch 2 h am Rückfluß engt ein und kristallisiert den Rückstand mit Petrolether.
Schmp. : 105-106 °C.

## Beispiel 34

Methyl-[3-(1-pyrrolidinyl)methyl-5-(1,1-dimethylethyl)-2-hydroxyphenyl] sulfoxid

Analog Beispiel 33, anstelle von Dimethylamin verwendet man Pyrrolidin.
Schmp. : 83-84 °C.

Beispiel 35

Methyl-[3-(4-Morpholinyl)methyl-5-(1,1-dimethylethyl)-2-hydroxyphenyl] sulfoxid

Analog Beispiel 33, anstelle von Dimethylamin verwendet man Morpholin.
Schmp. : 110-111 °C.

Beispiel 36

Methyl-[3-(3-thiazolidinyl)methyl-5-(1,1-dimethylethyl)-2-hydroxyphenyl] sulfoxid

Analog Beispiel 33, anstelle von Dimethylamin verwendet man Thiazolidin.
Schmp. : 164-165 °C.

Beispiel 37

2-Benzylaminomethyl-4-(1,1-dimethylethyl)-6-methylsulfonylphenol-hydrochlorid

a) [2-Hydroxy-3-methylsulfonyl-5-(1,1-dimethylethyl)benzyl] dimethylmethoxycarbonylammoniumchlorid

29,1 g (102 mMol) 2-Dimethylaminomethyl-4-(1,1-dimethylethyl)-6-methylsulfonylphenol werden in 250 ml Aceton gelöst und unter Eiskühlung mit 13,3 ml (108 mMol) Chlorameisensäuremethylester versetzt. Man beläßt 24 h bei Raumtemperatur und saugt den entstandenen Niederschlag ab.
Schmp. : 152-153 °C.

b) 2-Benzylaminomethyl-4-(1,1-dimethylethyl)-6-methylsulfonylphenol-hydrochlorid

9,85 g (25 mMol) des unter 36a) erhaltenen Ammoniumsalzes werden in 50 ml Methanol gelöst, mit 5,35 g (50 mMol) Benzylamin versetzt und 2 h unter Rückfluß gekocht. Man befreit vom Lösemittel, verreibt den Rückstand mit Ether, saugt ab und verrührt das Kristallisat mit 2n Salzsäure. Das sich abscheidende Öl kristallisiert.
Weiße Kristalle vom Schmp. : 213-215 °C.

Beispiel 38

2-(2-Phenylethylaminomethyl)-4-(1,1-dimethylethyl)-6-methylsulfonylphenol-hydrochlorid

Analog Beispiel 37b), jedoch mit 2-Phenylethylamin anstelle von Benzylamin. Das Rohprodukt wird aus Isopropanol umkristallisiert.
Schmp. : 195-197 °C.

Beispiel 39

2-Homoveratrylaminomethyl-4-(1,1-dimethylethyl)-6-methylsulfonylphenol-hydrochlorid

Analog Beispiel 37b), jedoch mit Homoveratrylamin anstelle von Benzylamin.
Schmp. : 216-218 °C.

Beispiel 40

2-Butylaminomethyl-4-(1,1-dimethylethyl)-6-methylsulfonylphenol-hydrochlorid

Analog Beispiel 37b), jedoch mit n-Butylamin anstelle von Benzylamin.
Schmp. : 140-141 °C.

Beispiel 41

2-Ethylaminomethyl-4-(1,1-dimethylethyl)-6-methylsulfonylphenol-hydrochlorid

Analog Beispiel 37b), jedoch mit Ethylamin anstelle von Benzylamin.
Schmp. : 187-189 °C.

Beispiel 42

2-Methylaminomethyl-4-(1,1-dimethylethyl)-6-methylsulfonylphenol-hydrochlorid

Analog Beispiel 37b) mit Methylamin anstelle von Benzylamin. Die Reaktionsmischung wird in einer geschlossenen Apparatur 4 h auf 150 °C gehalten.
Schmp. : 164-165 °C.

## Beispiel 43

2-Cyclohexylaminomethyl-4-(1,1-dimethylethyl)-6-methylsulfonylphenol-hydrochlorid

Analog Beispiel 37b) mit Cyclohexylamin anstelle von Benzylamin.
Schmp. : 210-211 °C.

## Beispiel 44

2-(1,1-Dimethylethyl)aminomethyl-4-(1,1-dimethylethyl)-6-methylsulfonylphenol-hydrochlorid

Analog Beispiel 42, jedoch mit tert. Butylamin anstelle von Methylamin.
Schmp. : 229-230 °C.

## Beispiel 45

2-(4-Methylbenzylaminomethyl)-4-(1,1-dimethylethyl)-6-methylsulfonylphenol-hydrochlorid

Analog Beispiel 37b), jedoch mit 4-Methylbenzylamin anstelle von Benzylamin.
Schmp. : 185-186 °C.

## Beispiel 46

2-(4-Methoxybenzylaminomethyl)-4-(1,1-dimethylethyl)-6-methylsulfonylphenol-hydrochlorid

Analog Beispiel 37b), jedoch mit 4-Methoxybenzylamin anstelle von Benzylamin.
Schmp. : 160-161 °C.

## Beispiel 47

2-(2-Chlorbenzylaminomethyl)-4-(1,1-dimethylethyl)-6-methylsulfonylphenol-hydrochlorid

Analog Beispiel 37b), jedoch mit 2-Chlorbenzylamin anstelle von Benzylamin.
Schmp. : 187-188 °C.

## Beispiel 48

2-(1-Phenylethylaminomethyl)-4-(1,1-dimethylethyl)-6-methylsulfonylphenol-hydrochlorid

Analog Beispiel 37b), jedoch mit 1-Phenylethylamin anstelle von Benzylamin.
Schmp. : 217-219 °C.

## Beispiel 49

2-[2-(4-Chlorphenyl)ethylaminomethyl]-4-(1,1-dimethylethyl)-6-methylsulfonylphenol-hydrochlorid

Analog Beispiel 37b), jedoch mit 2-(4-Chlorphenyl)ethylamin anstelle von Benzylamin.
Schmp. : 172-173 °C

## Beispiel 50

2-[2-(4-Methylphenyl)ethylaminomethyl]-4-(1,1-dimethylethyl)-6-methylsulfonylphenol-hydrochlorid

Analog Beispiel 37b), jedoch mit 2-(4-Methylphenyl)ethylamin anstelle von Benzylamin.
Schmp. : 157-158 °C

## Beispiel 51

2-Furfurylaminomethyl-4-(1,1-dimethylethyl)-6-methylsulfonylphenol-hydrochlorid

Analog Beispiel 37b), jedoch mit 2-Furfurylamin anstelle von Benzylamin.
Schmp. : 161-162 °C

Beispiel 52

4-Aminomethyl-1,1-dimethyl-5-hydroxy-6-methylsulfonyl-indan-hydrochlorid

a) 1,1-Dimethyl-5-methoxy-indan

100 g 1,1-Dimethyl-5-hydroxy-indan werden nach literaturbekannten Methoden (s. « Reaktion und Synthesen », L.F. Tieze, Th. Eicher, Thieme Verlag, Stuttgart 1981, S. 56) alkyliert.
Sdp. : 80-85 °C/2 mm Hg

b) 6-Chlorsulfonyl-1,1-dimethyl-5-methoxy-indan

Analog Beispiel 1a), jedoch mit 1,1-Dimethyl-5-methoxy-indan als Ausgangsmaterial. Das Produkt wird aus Diisopropylether umkristallisiert.
Kristalle vom Schmp. : 142-143 °C

c) 1,1-Dimethyl-5-methoxy-6-methylsulfonyl-indan

Analog Beispiel 1b) und 1c) jedoch mit 6-Chlorsulfonyl-1,1-dimethyl-5-methoxyindan als Ausgangsmaterial.
Das Produkt wird aus Diisopropylether umkristallisiert.
Kristalle vom Schmp. : 174-175 °C

d) 1,1-Dimethyl-5-hydroxy-6-methylsulfonyl-indan

Zu einer Lösung von 12,7 g (0,05 Mol) 1,1-Dimethyl-5-methoxy-6-methylsulfonyl-indan in 120 ml Methylenchlorid werden bei 0 °C 10 ml (0,1 Mol) Bortribromid zugetropft. Nach 4 h Rühren bei RT wird das überschüssige Bortribromid mit Methanol in der Kälte zerstört, die Lösung im Vakuum eingeengt und in Eiswasser eingetragen. Der Niederschlag wird nach dem Trocknen aus Diisopropylether umkristallisiert.
Kristalle vom Schmp. : 111-112 °C

e) 4-Chloracetamidomethyl-1,1-dimethyl-5-hydroxy-6-methylsulfonyl-indan

Zu einer Lösung von 9,6 g (0,04 Mol) 1,1-Dimethyl-5-hydroxy-6-methylsulfonyl-indan in einer Mischung aus 60 ml Eisessig und 30 ml konz. $H_2SO_4$ werden bei RT 7,4 g (0.06 Mol) Chloracetamidomethylol eingetragen, 1 h nachgerührt und in Eiswasser eingetragen. Der Niederschlag wird getrocknet und aus Essigsäureethylester umkristallisiert.
Kristalle vom Schmp. : 190-191 °C

f) 4-Aminomethyl-1,1-dimethyl-5-hydroxy-6-methylsulfonyl-indan-hydrochlorid

3,1 g (0,01 Mol) 4-Chloracetamidomethyl-1,1-dimethyl-5-hydroxy-6-methylsulfonyl-indan werden in 50 ml einer Mischung aus Ethanol/konz. HCl (4 : 1) 20 Stunden bei Rückflußtemperatur hydrolisiert. Anschließend wird eingeengt und der Rückstand aus Ethanol umkristallisiert.
Kristalle vom Schmp. : 246-248 °C.

**Patentansprüche** (für die Vertragsstaaten : BE, CH, DE, FR, GB, IT, LI, LU, NL, SE)

1. 2-Aminomethylphenole der Formel I

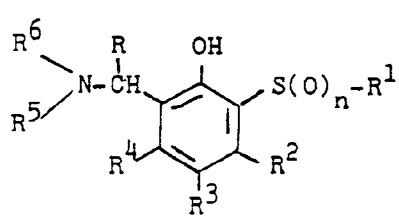

(I)

in welcher
R für Wasserstoff oder Alkyl mit 1 oder 2 C-Atomen,
$R^1$ für Alkyl mit 1 bis 8 C-Atomen, Cycloalkyl mit 3 bis 12 C-Atomen und bis zu 8 Ringgliedern, Alkenyl mit 2 bis 8 C-Atomen oder Cycloalkenyl mit 5 bis 12 C-Atomen und bis zu 8 Ringgliedern steht, wobei jeder

der vorstehend genannten Reste mit 1 bis 5 gleichen oder verschiedenen Halogenatomen substituiert sein kann,

$R^2$ und $R^4$ gleich oder verschieden sind und Wasserstoff, Halogen, Alkyl mit 1 oder 2 C-Atomen oder Alkoxy mit 1 oder 2 C-Atomen,

$R^3$ Halogen, Alkyl mit 1 bis 12 C-Atomen oder Cycloalkyl mit 3 bis 12 C-Atomen und bis zu 8 Ringgliedern bedeuten,

$R^5$ und $R^6$ gleich oder verschieden sind und für Wasserstoff, Alkyl mit 1 bis 6 C-Atomen, Cycloalkyl mit 3 bis 10 C-Atomen und bis zu 8 Ringgliedern oder den Rest $-[CH_2]_o-Ar$, worin o = 1 oder 2 ist, die $-[CH_2]_o-$ Kette durch 1 oder 2 ($C_1$ oder $C_2$)-Alkylgruppen substituiert sein kann und Ar ein gegebenenfalls durch 1 bis 3 gleiche oder verschiedene Reste ($C_1$ oder $C_2$)-Alkyl, ($C_1$ oder $C_2$)-Alkoxy oder Halogen substituiertes, 5- oder 6-gliedriges aromatisches oder heteroaromatisches System ist, stehen und

n 1 oder 2 ist,

wobei die Reste $R^5$ und $R^6$ und/oder zwei der Reste $R^2$, $R^3$ und $R^4$ auch eine $-[CH_2]_m-$ Kette mit m = 3 bis 6 bilden können, die gegebenenfalls durch 1 oder 2 Methylgruppen substituiert und die im Falle von $R^5$ und $R^6$ auch durch 1 oder 2 Sauerstoffatome, Schwefelatome und/oder Iminogruppen unterbrochen sein kann, sowie deren physiologisch verträglichen Salze.

2. Verbindungen der Formel I gemäß Anspruch 1, dadurch gekennzeichnet, daß

R Wasserstoff bedeutet,

$R^1$ für Alkyl mit 1 bis 4 C-Atomen, Cycloalkyl mit 3 bis 7 C-Atomen und bis zu 6 Ringgliedern, Alkenyl mit 2 bis 6 C-Atomen oder Halogenalkyl mit 1 bis 4 C-Atomen und 1 bis 3 gleichen oder verschiedenen Halogenatomen steht,

$R^3$ Halogen, Alkyl mit 1 bis 8 C-Atomen oder Cycloalkyl mit 3 bis 10 C-Atomen und bis zu 7 Ringgliedern bedeutet,

$R^5$ und $R^6$ die im Anspruch 1 definierten Bedeutungen haben mit der Ausnahme, daß $R^5$ und/oder $R^6$ für Alkyl mit 1 oder 2 C-Atomen steht,

n 1 oder 2 ist,

wobei die Reste $R^5$ und $R^6$ und/oder zwei der Reste $R^2$, $R^3$ und $R^4$ eine wie im Anspruch 1 definierte Polymethylenkette bilden können.

3. 2-Aminomethyl-4-(1,1-dimethylethyl)-6-methylsulfonylphenol sowie dessen physiologisch verträgliche Säureadditionssalze.

4. 2-Aminomethyl-4-(1,1-dimethylethyl)-6-chlormethylsulfonylphenol sowie dessen physiologisch verträgliche Säureadditionssalze.

5. 2-Aminomethyl-4-(1,1-dimethylpropyl)-6-methylsulfonylphenol sowie dessen physiologisch verträgliche Säureadditionssalze.

6. Methyl-[3-aminomethyl-5-(1,1-dimethylethyl)-2-hydroxyphenyl] sulfoxid sowie dessen physiologisch verträgliche Säureadditionssalze.

7. 2-Aminomethyl-4-isopropyl-6-methylsulfonylphenol sowie dessen physiologisch verträgliche Säureadditionssalze.

8. 2-Aminomethyl-4-(1,1-dimethylpropyl)-6-ethylsulfonylphenol sowie dessen physiologisch verträglichen Säureadditionssalze.

9. 2-Aminomethyl-4-(1,1-dimethylpropyl)-6-jodmethylsulfonyl-phenol sowie dessen physiologisch verträgliche Säureadditionssalze.

10. Verfahren zur Herstellung von 2-Aminomethylphenolen gemäß Anspruch 1, dadurch gekennzeichnet, daß man

a) Verbindungen der Formel II

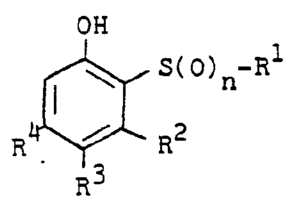

(II)

worin n, $R^1$, $R^2$, $R^3$ und $R^4$ die oben genannten Bedeutungen besitzen, mit einem N-Hydroxymethylcarboxamid der allgemeinen Formel III

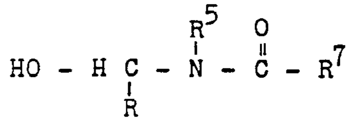

(III)

in der R vorstehende Bedeutung hat, $R^5$ Wasserstoff oder Alkyl mit 1 bis 4 C-Atomen bedeutet und $R^7$ für Wasserstoff, gegebenenfalls halogensubstituiertes Alkyl mit 1 bis 4 C-Atomen oder Aryl mit 6 bis 10 C-Atomen steht, oder in der $R^5$ und der Rest $COR^7$ gemeinsam für den o-Phthaloylrest stehen, zu Verbindungen der allgemeinen Formel IV umsetzt

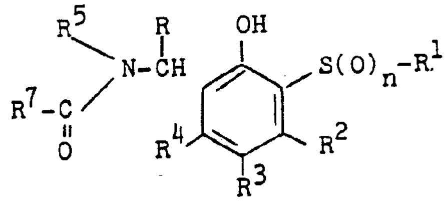

(IV)

und den Acylrest $R^7$—CO hydrolytisch abspaltet,

b) Verbindungen der Formel II, in der die Reste $R^1$ bis $R^4$ und n die oben erwähnten Bedeutungen haben, in Gegenwart von Formaldehyd mit Aminen der allgemeinen Formel V

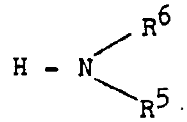

(V)

in der $R^5$ und $R^6$ die im Anspruch 1 genannten Bedeutungen mit Ausnahme von Wasserstoff besitzen, umsetzt

c) Verbindungen der allgemeinen Formel VI

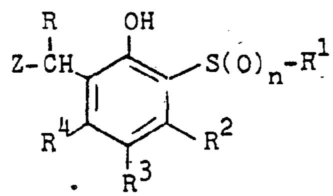

(VI)

in der n, R bis $R^4$ die oben genannten Bedeutungen haben und Z für eine Fluchtgruppe steht, mit Aminen der allgemeinen Formel H—N = Y, wobei Y für eine Aminschutzgruppe oder für die in Anspruch 1 definierten Reste $R^5$ und $R^6$ steht, wobei $R^6$ auch eine Aminschutzgruppe sein kann, zu Verbindungen der allgemeinen Formel VII umsetzt

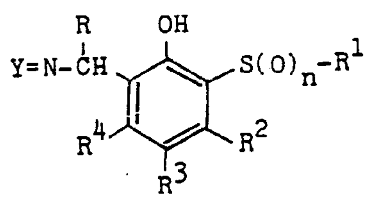

(VII)

und gegebenenfalls die Aminschutzgruppe hydrolytisch oder hydrogenolytisch abspaltet oder

d) Verbindungen der allgemeinen Formel XIV

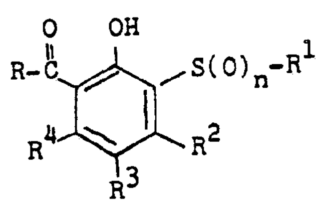

(XIV)

in der n und R bis $R^4$ die oben genannten Bedeutungen haben, umsetzt mit Aminen der Formel $R^5$—$NH_2$ mit $R^5$ in der oben genannten Bedeutung zu Schiffschen Basen der Formel XV

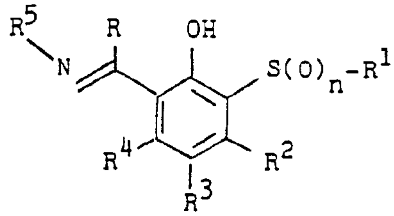

(XV)

in der n und R bis $R^5$ die oben genannten Bedeutungen haben und diese zu Verbindungen der Formel I reduziert und die nach einer der Varianten a)-d) erhaltenen Verbindungen der Formel I gegebenenfalls in ihre physiologisch verträglichen Salze überführt.

11. Pharmazeutische Präparate auf Basis einer Verbindung gemäß Anspruch 1.

12. Verfahren zur Herstellung eines pharmazeutischen Praparates gemäß Anspruch 11, dadurch gekennzeichnet, daß man eine Verbindung gemäß Anspruch 1 gegebenenfalls mit pharmakologisch verträglichen Trägern und/oder Stabilisatoren in eine geeignete Darreichungsform überführt.

13. Verbindung der Formel I nach Anspruch 1 zur Verwendung als Diuretikum und Antihypertonikum.

14. Verbindungen der allgemeinen Formel II

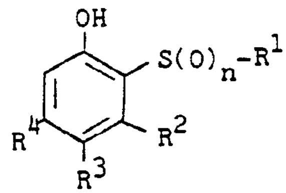

(II)

in welcher n, $R^1$ bis $R^4$ die in Anspruch 1 angegebenen Bedeutungen haben.

**Patentansprüche** (für den Vertragsstaat AT)

1. Verfahren zur Herstellung von 2-Aminomethylphenolen der Formel I

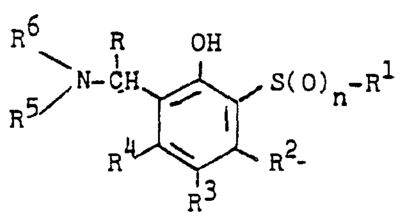

(I)

in welcher

R Wasserstoff oder Alkyl mit 1 oder 2 C-Atomen,

$R^1$ für Alkyl mit 1 bis 8 C-Atomen, Cycloalkyl mit 3 bis 12 C-Atomen und bis zu 8 Ringgliedern, Alkenyl mit 2 bis 8 C-Atomen oder Cycloalkenyl mit 5 bis 12 C-Atomen und bis zu 8 Ringgliedern steht, wobei jeder der vorstehend genannten Reste mit 1 bis 5 gleichen oder verschiedenen Halogenatomen substituiert sein kann,

$R^2$ und $R^4$ gleich oder verschieden sind und Wasserstoff, Halogen, Alkyl mit 1 oder 2 C-Atomen oder Alkoxy mit 1 oder 2 C-Atomen,

$R^3$ Halogen, Alkyl mit 1 bis 12 C-Atomen oder Cycloalkyl mit 3 bis 12 C-Atomen und bis zu 8 Ringgliedern bedeuten,

$R^5$ und $R^6$ gleich oder verschieden sind und für Wasserstoff, Alkyl mit 1 bis 6 C-Atomen, Cycloalkyl mit 3 bis 10 C-Atomen und bis zu 8 Ringgliedern oder den Rest $-[CH_2]_o-Ar$, worin o = 1 oder 2 ist, die $-[CH_2]_o-$ Kette durch 1 oder 2 ($C_1$ oder $C_2$)-Alkylgruppen substituiert sein kann und Ar ein gegebenenfalls durch 1 bis 3 gleiche oder verschiedene Reste ($C_1$ oder $C_2$)-Alkyl, ($C_1$ oder $C_2$)-Alkoxy oder Halogen substituiertes, 5- oder 6-gliedriges aromatisches oder heteroaromatisches System ist, stehen und

n 1 oder 2 ist,

wobei die Reste $R^5$ und/oder zwei der Reste $R^2$, $R^3$ und $R^4$ auch eine $-[CH_2]_m-$ Kette mit m = 3 bis 6 bilden können, die gegebenenfalls durch 1 oder 2 Methylgruppen substituiert und die im Falle von $R^5$ und $R^6$ auch durch 1 oder 2 Sauerstoffatome, Schwefelatome und/oder Iminogruppen unterbrochen sein kann, sowie deren physiologisch verträglichen Salze, dadurch gekennzeichnet, daß man

a) Verbindungen der Formel II

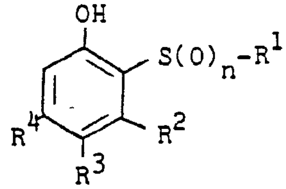

(II)

worin n, $R^1$, $R^2$, $R^3$ und $R^4$ die oben genannten Bedeutungen besitzen, mit einem N-Hydroxymethylcarboxamid der allgemeinen Formel III

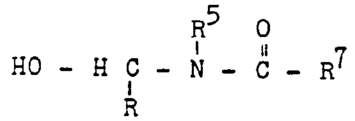

(III)

in der R vorstehende Bedeutung hat, $R^5$ Wasserstoff oder Alkyl mit 1 bis 4 C-Atomen bedeutet und $R^7$ für Wasserstoff, gegebenenfalls halogensubstituiertes Alkyl mit 1 bis 4 C-Atomen oder Aryl mit 6 bis 10 C-Atomen steht, oder in der $R^5$ und der Rest $COR^7$ gemeinsam für den o-Phthaloylrest stehen, zu Verbindungen der allgemeinen Formel IV umsetzt

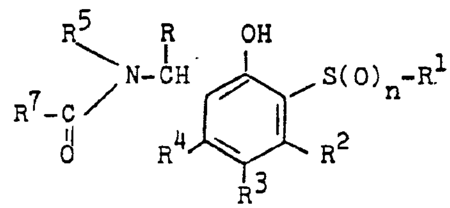

(IV)

und den Acylrest $R^7$—CO hydrolytisch abspaltet,

b) Verbindungen der Formel II, in der die Reste $R^1$ bis $R^4$ und n die oben erwähnten Bedeutungen haben, in Gegenwart von Formaldehyd mit Aminen der allgemeinen Formel V

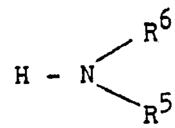

(V)

in der $R^5$ und $R^6$ die im Anspruch 1 genannten Bedeutungen mit Ausnahme von Wasserstoff besitzen, umsetzt,

c) Verbindungen der allgemeinen Formel VI

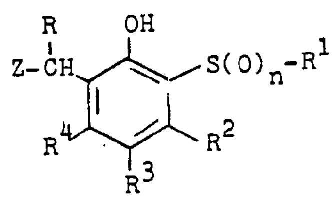

(VI)

in der n, R bis $R^4$ die oben genannten Bedeutungen haben und Z für eine Fluchtgruppe steht, mit Aminen der allgemeinen Formel H—N = Y, wobei Y für eine Aminschutzgruppe oder für die in Anspruch 1 definierten Reste $R^5$ und $R^6$ steht, wobei $R^6$ auch eine Aminschutzgruppe sein kann, zu Verbindungen der allgemeinen Formel VII umsetzt

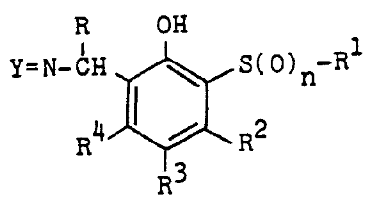

(VII)

und gegebenenfalls die Aminschutzgruppe hydrolytisch oder hydrogenolytisch abspaltet oder

d) Verbindungen der allgemeinen Formel XIV

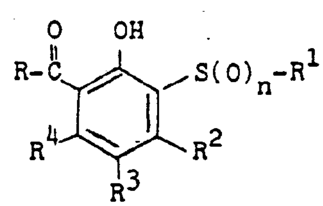

(XIV)

in der n und R bis $R^4$ die oben genannten Bedeutungen haben, umsetzt mit Aminen der Formel $R^5$—$NH_2$ mit $R^5$ in der oben genannten Bedeutung zu Schiffschen Basen der Formel XV

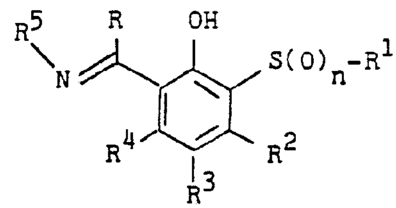

(XV)

in der n und R bis $R^5$ die oben genannten Bedeutungen haben und diese zu Verbindungen der Formel I reduziert, und die nach einer der Varianten a)-d) erhaltenen Verbindungen der Formel I gegebenenfalls in ihre physiologisch verträglichen Salze überführt.

2. Verfahren gemäß Anspruch 1, dadurch gekennzeichnet, daß

R Wasserstoff bedeutet,

$R^1$ für Alkyl mit 1 bis 4 C-Atomen, Cycloalkyl mit 3 bis 7 C-Atomen und bis zu 6 Ringgliedern, Alkenyl mit 2 bis 6 C-Atomen oder Halogenalkyl mit 1 bis 4 C-Atomen und 1 bis 3 gleichen oder verschiedenen Halogenatomen steht,

$R^3$ Halogen, Alkyl mit 1 bis 8 C-Atomen oder Cycloalkyl mit 3 bis 10 C-Atomen und bis zu 7 Ringgliedern bedeutet,

$R^5$ und $R^6$ die im Anspruch 1 definierten Bedeutungen haben mit der Ausnahme, daß $R^5$ und/oder $R^6$ für Alkyl mit 1 oder 2 C-Atomen steht,

n 1 oder 2 ist,

wobei die Reste $R^5$ und $R^6$ und/oder zwei der Reste $R^2$, $R^3$ und $R^4$ eine wie im Anspruch 1 definierte Polymethylenkette bilden können.

3. Verfahren gemäß Anspruch 1, dadurch gekennzeichnet, daß 2-Aminomethyl-4-(1,1-dimethylethyl)-6-methyl-sulfonylphenol hergestellt und gegebenenfalls in sein physiologisch verträgliches Säureadditionssalze überführt wird.

4. Verfahren gemäß Anspruch 1, dadurch gekennzeichnet, daß 2-Aminomethyl-4-(1,1-dimethylethyl)-6-chlormethylsulfonylphenol hergestellt und gegebenenfalls in sein physiologisch verträgliches Säureadditionssalz überführt wird.

5. Verfahren gemäß Anspruch 1, dadurch gekennzeichnet, daß 2-Aminomethyl-4-(1,1-dimethylpropyl)-6-methylsulfonylphenol hergestellt und gegebenenfalls in sein physiologisch verträgliches Säureadditionssalz überführt wird.

6. Verfahren gemäß Anspruch 1, dadurch gekennzeichnet, daß Methyl-[3-aminomethyl-5-(1,1-dimethylethyl)-2-hydroxyphenyl] sulfoxid hergestellt und gegebenenfalls in sein physiologisch verträgliches Säureadditionssalz überführt wird.

7. Verfahren gemäß Anspruch 1, dadurch gekennzeichnet, daß 2-Aminomethyl-4-isopropyl-6-methylsulfonylphenol hergestellt und gegebenenfalls in sein physiologisch verträgliches Säureadditionssalz überführt wird.

8. Verfahren gemäß Anspruch 1, dadurch gekennzeichnet, daß 2-Aminomethyl-4-(1,1-dimethylpropyl)-6-ethylsulfonylphenol hergestellt und gegebenenfalls in sein physiologisch verträgliches Säureadditionssalz überführt wird.

9. Verfahren gemäß Anspruch 1, dadurch gekennzeichnet, daß 2-Aminomethyl-4-(1,1-dimethylpropyl)-6-jodmethylsulfonyl-phenol hergestellt und gegebenenfalls in sein physiologisch verträgliches Säureadditionssalz überführt wird.

10. Pharmazeutische Präparate auf Basis einer Verbindung gemäß Anspruch 1.

11. Verfahren zur Herstellung eines pharmazeutischen Präparates gemäß Anspruch 8, dadurch gekennzeichnet, daß man ein Verbindung gemäß Anspruch 1 gegebenenfalls mit pharmakologisch verträglichen Trägern und/oder Stabilisatoren in eine geeignete Darreichungsform überführt.

12. Verfahren zur Herstellung von Verbindungen der allgemeinen Formel II

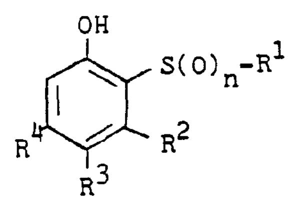

(II)

in welcher n, $R^1$ bis $R^4$ die in Anspruch 1 angegebenen Bedeutungen haben, dadurch gekennzeichnet, daß man Thioether der Formel VIII

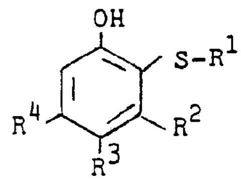

(VIII)

in der $R^1$ bis $R^4$ die obige Bedeutung haben, oxidiert.

**Claims** (for the Contracting States : BE, CH, DE, FR, GB, IT, LI LU, NL, SE)

1. 2-Aminomethylphenols of the formula I

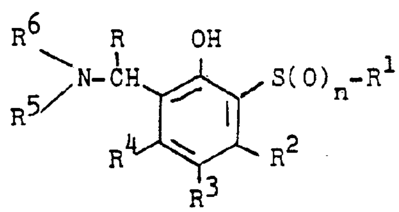

(I)

in which

R represents hydrogen or alkyl having 1 or 2 C atoms, $R^1$ represents alkyl having 1 to 8 C atoms, cycloalkyl having 3 to 12 C atoms and up to 8 ring members, alkenyl having 2 to 8 C atoms or cycloalkenyl having 5 to 12 C atoms and up to 8 ring members, it being possible for each of the aforementioned radicals to be substituted with 1 to 5 identical or different halogen atoms,

$R^2$ and $R^4$ are identical or different and denote hydrogen, halogen, alkyl having 1 or 2 C atoms or alkoxy having 1 or 2 C atoms,

$R^3$ denotes halogen, alkyl having 1 to 12 C atoms or cycloalkyl having 3 to 12 C atoms and up to 8 ring members,

$R^5$ and $R^6$ are identical or different and represent hydrogen, alkyl having 1 to 6 C atoms, cycloalkyl having 3 to 10 C atoms and up to 8 ring members or the radical $-[CH_2]_o-Ar$, wherein o = 1 or 2, the $-[CH_2]_o-$ chain can be substituted by 1 or 2 ($C_1$ or $C_2$)-alkyl groups and Ar is a 5- or 6- membered aromatic or heteroaromatic system, which is optionally substituted by 1 to 3 identical or different radicals ($C_1$ or $C_2$)-alkyl, ($C_1$ or $C_2$)-alkoxy or halogen, and n is 1 or 2,

it being possible for the radicals $R^5$ and $R^6$ and/or two of the radicals $R^2$, $R^3$ and $R^4$ also to form a $-[CH_2]_m-$ chain having m = 3 to 6, which can optionally be substituted by 1 or 2 methyl groups and, in the case of $R^5$ and $R^6$, can also be interrupted by 1 or 2 oxygen atoms, sulfur atoms and/or imino groups, and their physiologically tolerated salts.

2. A compound of the formula I as claimed in claim 1, in which

R denotes hydrogen,

$R^1$ represents alkyl having 1 to 4 C atoms, cycloalkyl having 3 to 7 C atoms and up to 6 ring members, alkenyl having 2 to 6 C atoms or halogenoalkyl having 1 to 4 C atoms and 1 to 3 identical or different halogen atoms,

$R^3$ denotes halogen, alkyl having 1 to 8 C atoms or cycloalkyl having 3 to 10 C atoms and up to 7 ring members,

$R^5$ and $R^6$ have the meanings defined in claim 1 with the exception that $R^5$ and/or $R^6$ represent alkyl having 1 or 2 C atoms,

n is 1 or 2,

it being possible for the radicals $R^5$ and $R^6$ and/or two of the radicals $R^2$, $R^3$ and $R^4$ to form a polymethylene chain as defined in claim 1.

3. 2-Aminomethyl-4-(1,1-dimethylethyl)-6-methylsulfonylphenol and its physiologically tolerated acid addition salts.

4. 2-Aminomethyl-4-(1,1-dimethylethyl)-6-chloromethylsulfonylphenol and its physiologically tolerated acid addition salts.

5. 2-Aminomethyl-4-(1,1-dimethylpropyl)-6-methylsulfonylphenol and its physiologically tolerated acid addition salts.

6. Methyl [3-aminomethyl-5-(1,1-dimethylethyl)-2-hydroxyphenyl] sulfoxide and its physiologically tolerated acid addition salts.

7. 2-Aminomethyl-4-isopropyl-6-methylsulfonylphenol and its physiologically tolerated acid addition salts.

8. 2-Aminomethyl-4-(1,1-dimethylpropyl)-6-ethylsulfonylphenol and its physiologically tolerated acid addition salts.

9. 2-Aminomethyl-4-(1,1-dimethylpropyl)-6-idiomethylsulfonylphenol and its physiologically tolerated acid addition salts.

10. A process for the preparation of the 2-aminomethylphenols as claimed in claim 1, which comprises

a) reacting a compound of the formula II

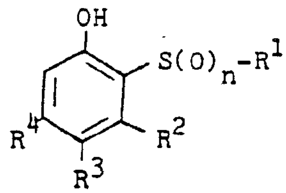

(II)

wherein n, $R^1$, $R^2$, $R^3$ and $R^4$ have the abovementioned meanings, with an N-hydroxymethylcarboxamide of the general formula III

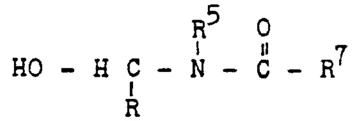

(III)

in which R has the abovementioned meaning, $R^5$ denotes hydrogen or alkyl having 1 to 4 C atoms and $R^7$ represents hydrogen, optionally halogen-substituted alkyl having 1 to 4 C atoms or aryl having 6 to 10 C atoms, or in which $R^5$ and the radical $COR^7$ together represent the o-phthaloyl radical, to give a compound of the general formula IV

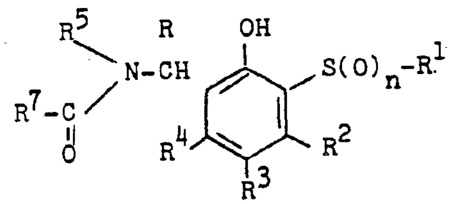

(IV)

and removing the acyl radical $R^7$-CO by hydrolysis,

b) reacting a compound of the formula II, in which the radicals $R^1$ to $R^4$ and n have the abovementioned meanings, in the presence of formaldehyde, with an amine of the general formula V

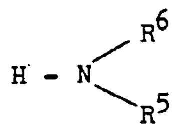

(V)

in which $R^5$ and $R^6$ have the meanings mentioned in claim 1 with the exception of hydrogen,

c) reacting a compound of the general formula VI

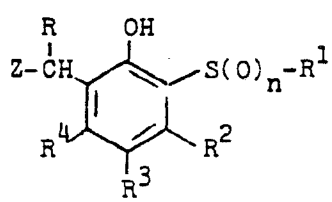

(VI)

in which n and R to $R^4$ have the abovementioned meanings and Z represents a leaving group, with an amine of the general formula H—N = Y, wherein Y represents an amine-protective group or the radicals $R^5$ and $R^6$ defined in claim 1, it being possible for $R^6$ also to be an amine-protective group, to give a compound of the general formula VII

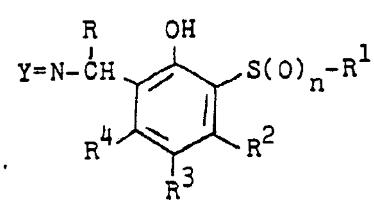

(VII)

and, if appropriate, removing the amine-protective group by hydrolysis or hydrogenolysis, or

d) reacting compounds of the general formula XIV

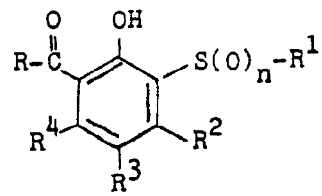

(XIV)

in which n and R to $R^4$ have the abovementioned meanings, with an amine of the formula $R^5$—$NH_2$, with $R^5$ having the abovementioned meaning, to give Schiff's bases of the formula XV

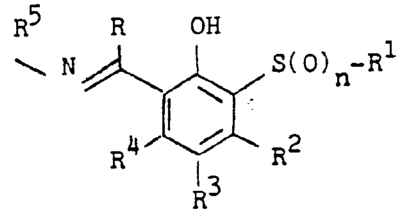

(XV)

in which n and R to $R^5$ have the abovementioned meanings, and reducing the latter to give a compound of the formula I, and optionally converting the compound of the formula I obtained according to one of the variants a)-d) into its physiologically tolerated salts.

11. A pharmaceutical formulation based on a compound as claimed in claim 1.

12. A process for the preparation of a pharmaceutical formulation as claimed in claim 11, which comprises converting a compound as claimed in claim 1, optionally with pharmacologically tolerated vehicles and/or stabilizers, into a suitable form for administration.

13. A compound as claimed in claim 1 for use as diuretic or antihypertensivum.

14. A compound of the general formula II

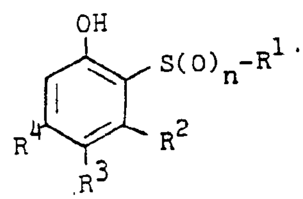

(II)

in which n, $R^1$ to $R^4$ have the meanings mentioned in claim 1.

**Claims** (for the Contracting State AT)

1. A process for the preparation of the 2-aminomethylphenols of the formula I

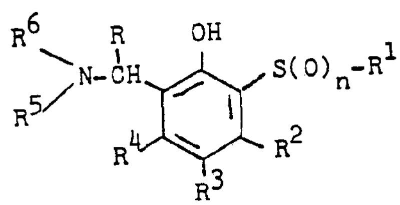

(I)

in which

R represents hydrogen or alkyl having 1 or 2 C atoms,

$R^1$ represents alkyl having 1 to 8 C atoms, cycloalkyl having 3 to 12 C atoms and up to 8 ring members, alkenyl having 2 to 8 C atoms or cycloalkenyl having 5 to 12 C atoms and up to 8 ring members, it being possible for each of the aforementioned radicals to be substituted with 1 to 5 identical or different halogen atoms,

$R^2$ and $R^4$ are identical or different and denote hydrogen, halogen, alkyl having 1 or 2 C atoms or alkoxy having 1 or 2 C atoms,

$R^3$ denotes halogen, alkyl having 1 to 12 C atoms or cycloalkyl having 3 to 12 C atoms and up to 8 ring members,

$R^5$ and $R^6$ are identical or different and represent hydrogen, alkyl having 1 to 6 C atoms, cycloalkyl having 3 to 10 C atoms and up to 8 ring members or the radical $—[CH_2]_o—Ar$, wherein o = 1 or 2, the $—[CH_2]_o—$ chain can be substituted by 1 or 2 ($C_1$ or $C_2$)-alkyl groups and Ar is a 5- or 6-membered aromatic or heteroaromatic system, which is optionally substituted by 1 to 3 identical or different radicals ($C_1$ or $C_2$)-alkyl, ($C_1$ or $C_2$)-alkoxy or halogen, and

n is 1 or 2,

it being possible for the radicals $R^5$ and $R^6$ and/or two of the radicals $R^2$, $R^3$ and $R^4$ also to form a $—[CH_2]_m—$ chain having m = 3 to 6, which can optionally be substituted by 1 or 2 methyl groups and, in the case of $R^5$ and $R^6$, can also be interrupted by 1 or 2 oxygen atoms, sulfur atoms and/or imino groups, and their physiologically tolerated salts, which comprises

a) reacting a compound of the formula II

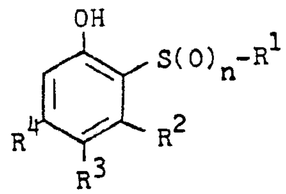

(II)

wherein n, $R^1$, $R^2$, $R^3$ and $R^4$ have the abovementioned meanings, with an N-hydroxymethylcarboxamide of the general formula III

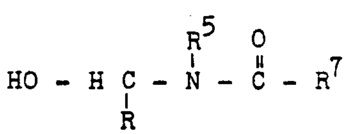

(III)

in which R has the abovementioned meaning, $R^5$ denotes hydrogen or alkyl having 1 to 4 C atoms and $R^7$ represents hydrogen, optionally halogen-substituted alkyl having 1 to 4 C atoms or aryl having 6 to 10 C atoms, or in which $R^5$ and the radical $COR^7$ together represent the o-phthaloyl radical, to give a compound of the general formula IV

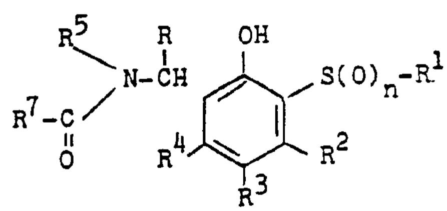

(IV)

and removing the acyl radical $R^7—CO$ by hydrolysis,

b) reacting a compound of the formula II, in which the radicals $R^1$ to $R^4$ and n have the abovementioned meanings, in the presence of formaldehyde, with an amine of the general formula V

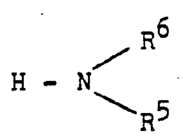

(V)

in which $R^5$ and $R^6$ have the meanings mentioned in claim 1 with the exception of hydrogen,

c) reacting a compound of the general formula VI

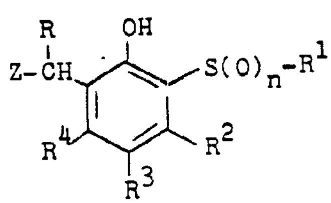

(VI)

in which n and R to $R^4$ have the abovementioned meanings and Z represents a leaving group, with an amine of the general formula H—N = Y, wherein Y represents an amine-protective group or the radicals

$R^5$ and $R^6$ defined in claim 1, it being possible for $R^6$ also to be an amine-protective group, to give a compound of the general formula VII

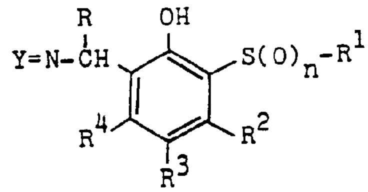

(VII)

and, if appropriate, removing the amine-protective group by hydrolysis or hydrogenolysis, or
d) reacting compounds of the general formula XIV

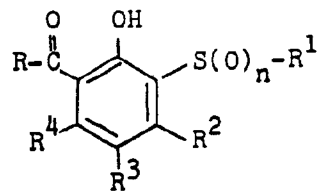

(XIV)

in which n and R to $R^4$ have the abovementioned meanings, with an amine of the formula $R^5$—$NH_2$, with $R^5$ having the abovementioned meaning, to give Schiff's bases of the formula XV

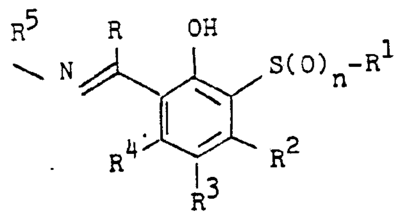

(XV)

in which n and R to $R^5$ have the abovementioned meanings, and reducing the latter to give a compound of the formula I, and optionally converting the compound of the formula I obtained according to one of the variants a) — d) into its physiologically tolerated salts.

2. A process according to claim 1, in which
R denotes hydrogen,
$R^1$ represents alkyl having 1 to 4 C atoms, cycloalkyl having 3 to 7 C atoms and up to 6 ring members, alkenyl having 2 to 6 C atoms or halogenoalkyl having 1 to 4 C atoms and 1 to 3 identical or different halogen atoms,
$R^3$ denotes halogen, alkyl having 1 to 8 C atoms or cycloalkyl having 3 to 10 C atoms and up to 7 ring members,
$R^5$ and $R^6$ have the meanings defined in claim 1 with the exception that $R^5$ and/or $R^6$ represent alkyl having 1 or 2 C atoms,
n is 1 or 2,
it being possible for the radicals $R^5$ and $R^6$ and/or two of the radicals $R^2$, $R^3$ and $R^4$ to form a polymethylene chain as defined in claim 1.

3. Process according to claim 1, in which 2-amino-methyl-4-(1,1-dimethylethyl)-6-methyl-sulfonyl-phenol is prepared and is optionally converted into its physiologically tolerated acid addition salt.

4. Process according to claim 1, in which 2-amino-methyl-4-(1,1-dimethylethyl)-6-chloro-methylsulfonylphenol is prepared and optionally converted into its physiologically tolerated acid addition salt.

5. Process according to claim 1, in which 2-aminomethyl-4-(1,1-dimethyl propyl)-6-methylsulfonyl-phenol is prepared and optionally converted into its physiologically tolerated acid addition salt.

6. A process according to claim 1, in which methyl [3-aminomethyl-5-(1,1-dimethylethyl)-2-hydroxyphenyl] sulfoxide is prepared and its optionally converted into its physiologically tolerated acid addition salt.

7. A process according to claim 1, in which 2-amino-methyl-4-isopropyl-6-methylsulfonylphenol is prepared and is optionally converted into its physiologically tolerated acid addition salt.

8. A process according to claim 1, in which 2-amino-methyl-4-(1,1-dimethylpropyl)-6-ethylsulfonyl-phenol is prepared and is optionally converted into its physiologically tolerated acid addition salt.

9. A process according to claim 1, in which 2-amino-methyl-4-(1,1-dimethylpropyl)-6-iodomethylsulfonylphenol is prepared and is optionally converted into its physiologically tolerated acid addition salt.

10. A pharmaceutical formulation based on a compound as claimed in claim 1.

11. A process for the preparation of a pharmaceutical formulation as claimed in claim 10, which comprises converting a compound as claimed in claim 1, optionally with pharmacologically tolerated vehicles and/or stabilizers, into a suitable form for administration.

12. A process for the production of compounds of the general formula II

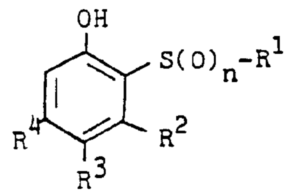

(II)

in which n, $R^1$ to $R^4$ have the meanings mentioned in claim 1, which comprises oxidizing thioethers of formula VIII

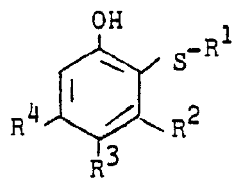

(VIII)

in which $R^1$ to $R^4$ have the above mentioned meanings.

**Revendications** (pour les Etats contractants : BE, CH, DE, FR, GB, IT, LI, LU, NL, SE)

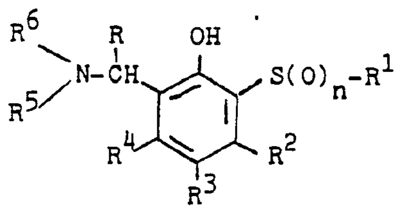

(I)

dans laquelle

R représente l'hydrogène ou un groupe alcoyle ayant 1 ou 2 atomes de carbone,

$R^1$ représente un groupe alcoyle ayant de 1 à 8 atomes de carbone, cycloalcoyle ayant de 3 à 12 atomes de carbone et jusqu'à 8 chaînons dans le cycle, alcényle ayant de 2 à 8 atomes de carbone ou cycloalcényle ayant de 5 à 12 atomes de carbone et jusqu'à 8 chaînons dans le cycle, chacun des radicaux mentionnés ci-dessus pouvant être substitué avec de 1 à 5 atomes d'halogène identiques ou différents,

$R^2$ et $R^4$ sont identiques ou différents et représentent l'hydrogène, un halogène, un groupe alcoyle ayant 1 ou 2 atomes de carbone ou alcoxy ayant 1 ou 2 atomes de carbone,

$R^3$ représente un halogène, un groupe alcoyle ayant de 1 à 12 atomes de carbone ou cycloalcoyle ayant de 3 à 12 atomes de carbone et jusqu'à 8 chaînons dans le cycle,

$R^5$ et $R^6$ sont identiques ou différents et représentent l'hydrogène, un groupe alcoyle ayant de 1 à 6 atomes de carbone, cycloalcoyle ayant de 3 à 10 atomes de carbone et jusqu'à 8 chaînons dans le cycle ou le radical $-[CH_2]_o-Ar$ dans lequel o = 1 ou 2, la chaîne $-[CH_2]_o-$ peut être substituée par 1 ou 2 groupes alcoyle en $C_1$ ou $C_2$ et Ar est un système aromatique ou hétéro-aromatique à 5 ou 6 chaînons éventuellement substitué par de 1 à 3 groupes alcoyle en $C_1$ ou $C_2$, alcoxy en $C_1$ ou $C_2$ ou halogènes identiques ou différents, et

n est 1 ou 2,

les radicaux $R^5$ et $R^6$ et/ou deux des radicaux $R^2$, $R^3$, $R^4$ pouvant également former une chaîne $-[CH_2]_m-$ avec m = de 3 à 6, qui peut être éventuellement substituée par 1 ou 2 groupes méthyle et qui, dans le cas de $R^5$ et $R^6$, peut également être interrompue par 1 ou 2 atomes d'oxygène, de soufre et/ou groupes imino, et leurs sels physiologiquement acceptables.

2. Composés de formule I selon la revendication 1, caractérisés en ce que :

R représente l'hydrogène,

$R^1$ représente un groupe alcoyle ayant de 1 à 4 atomes de carbone, cycloalcoyle ayant de 3 à 7 atomes de carbone et jusqu'à 6 chaînons dans le cycle, alcényle ayant de 2 à 6 atomes de carbone ou halogénoalcoyle ayant de 1 à 4 atomes de carbone et de 1 à 3 atomes d'halogène identiques ou différents,

$R^3$ représente un halogène, un groupe alcoyle ayant de 1 à 8 atomes de carbone ou cycloalcoyle ayant de 3 à 10 atomes de carbone et jusqu'à 7 chaînons dans le cycle,

$R^5$ et $R^6$ ont les significations définies dans la revendication 1, si ce n'est que $R^5$ et/ou $R^6$ ne représentent pas un groupe alcoyle ayant 1 ou 2 atomes de carbone,

n est 1 ou 2

les radicaux $R^5$ et $R^6$ et/ou deux des radicaux $R^2$, $R^3$ et $R^4$ pouvant former une chaîne polyméthylène telle que définie dans la revendication 1.

3. 2-aminométhyl-4-(1,1-diméthyléthyl)-6-méthylsulfonylphénol ainsi que ses sels d'addition avec des acides, physiologiquement acceptables.

4. 2-aminométhyl-4-(1,1-diméthyléthyl)-6-chlorométhylsulfonylphénol ainsi que ses sels d'addition avec des acides physiologiquement acceptables.

5. 2-aminométhyl-4-(1,1-diméthylpropyl)-6-méthyl-sulfonylphénol ainsi que ses sels d'addition avec des acides, physiologiquement acceptables.

6. Méthyl-[3-aminométhyl-5-(1,1-diméthyléthyl)-2-hydroxyphényl] sulfoxyde ainsi que ses sels d'addition avec des acides, physiologiquement acceptables.

7. 2-aminométhyl-4-isopropyl-6-méthylsulfonylphénol ainsi que ses sels d'addition avec des acides, physiologiquement acceptables.

8. 2-aminométhyl-4-(1,1-diméthylpropyl)-6-éthylsulfonylphénol ainsi que ses sels d'addition avec des acides, physiologiquement acceptables.

9. 2-aminométhyl-4-(1,1-diméthylpropyl)-6-iodométhyl-sulfonylphénol ainsi que ses sels d'addition avec des acides, physiologiquement acceptables.

10. Procédé de préparation de 2-aminométhylphénols selon la revendication 1, caractérisé en ce qu'il consiste

a) à faire réagir des composés de formule II

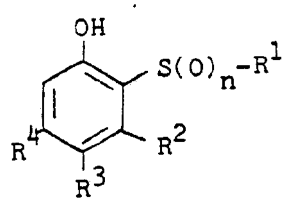

(II)

dans laquelle n, $R^1$, $R^2$, $R^3$ et $R^4$ ont les significations indiquées ci-dessus, avec un N-hydroxyméthylcarboxamide de formule générale III

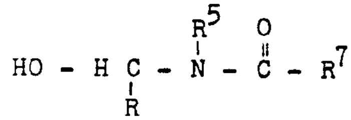

(III)

dans laquelle R a la signification ci-dessus, $R^5$ représente l'hydrogène ou un groupe alcoyle ayant de 1 à 4 atomes de carbone et $R^7$ représente l'hydrogène, un groupe alcoyle ayant de 1 à 4 atomes de carbone, éventuellement substitué par un halogène, ou un groupe aryle ayant de 6 à 10 atomes de carbone, ou dans laquelle $R^5$ et le radical $COR^7$ représentent ensemble un radical o-phtaloyle, pour obtenir des composés de formule générale IV

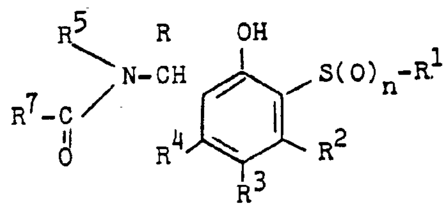

(IV)

et à éliminer le groupe acyle $R^7$—CO par hydrolyse,

b) à faire réagir des composés de formule II dans lesquels les radicaux $R^1$ à $R^4$ et n ont les significations indiquées ci-dessus, en présence de formaldéhyde, avec des amines de formule générale V

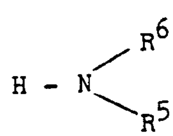

(V)

dans laquelle $R^5$ et $R^6$ ont les significations indiquées dans la revendication 1, à l'exception de l'hydrogène,

c) à faire réagir des composés de formule générale VI

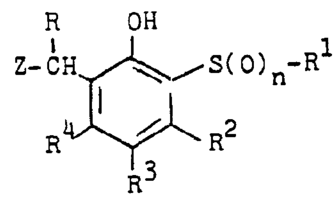

(VI)

dans laquelle n, R à $R^4$ ont les significations indiquées ci-dessus et Z représente un groupe labile, avec des amines de formule générale H—N = Y dans laquelle Y représente un groupe amino-protecteur ou les radicaux $R^5$ et $R^6$ définis dans la revendication 1, $R^6$ pouvant être également un groupe amino-protecteur, pour obtenir des composés de formule générale VII

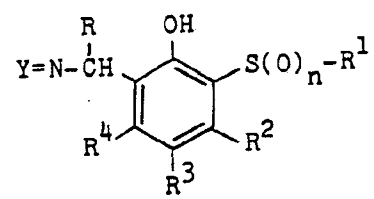

(VII)

et à éliminer éventuellement le groupe amino-protecteur par hydrolyse ou hydrogénolyse ·

d) à faire réagir des composés de formule générale XIV

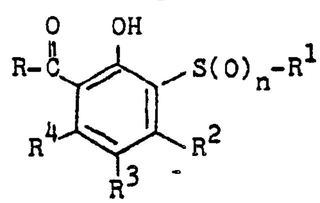

(XIV)

dans laquelle n et R à $R^4$ ont les significations indiquées ci-dessus, avec des amines de formule $R^5$—$NH_2$, $R^5$ ayant la signification indiquée ci-dessus, pour obtenir des bases de Schiff de formule XV

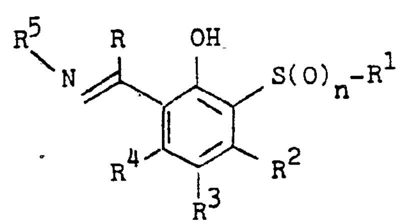

(XV)

dans laquelle n et R à $R^5$ ont les significations ci-dessus et à réduire ces dernières en composés de formule I et à convertir éventuellement les composés de formule I obtenus selon les variantes a) à d) en leurs sels physiologiquement acceptables.

11. Compositions pharmaceutiques contenant un composé selon la revendication 1.

12. Procédé de préparation d'une composition pharmaceutique selon la revendication 11, caractérisé en ce qu'on convertit un composé selon la revendication 1, éventuellement avec des véhicules et/ou des stabilisants pharmacologiquement acceptables, en une forme d'administration appropriée.

13. Composé de formule I selon la revendication 1, pour utilisation comme diurétique et antihypertonique.

14. Composé de formule générale II

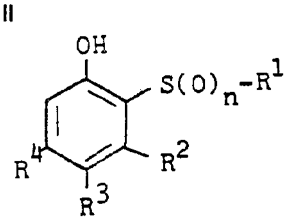

(II)

dans laquelle n, $R^1$ à $R^4$ ont les significations indiquées dans la revendication 1.

**Revendications** (pour l'Etat contractant AT)

1. Procédé de préparation de 2-aminométhylphénols de formule I

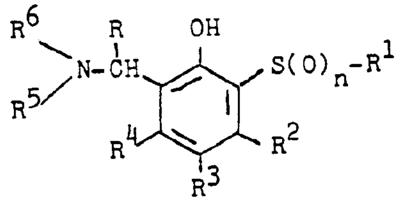

(I)

dans laquelle

R représente l'hydrogène ou un groupe alcoyle ayant 1 ou 2 atomes de carbone,

$R^1$ représente un groupe alcoyle ayant de 1 à 8 atomes de carbone, cycloalcoyle ayant de 3 à 12 atomes de carbone et jusqu'à 8 chaînons dans le cycle, alcényle ayant de 2 à 8 atomes de carbone ou cycloalcényle ayant de 5 à 12 atomes de carbone et jusqu'à 8 chaînons dans le cycle, chacun des radicaux mentionnés ci-dessus pouvant être substitué avec de 1 à 5 atomes d'halogène identiques ou différents,

$R^2$ et $R^4$ sont identiques ou différents et représentent l'hydrogène, un halogénure, un groupe alcoyle ayant 1 ou 2 atomes de carbone ou alcoxy ayant 1 ou 2 atomes de carbone,

$R^3$ représente un halogène, un groupe alcoyle ayant de 1 à 12 atomes de carbone ou cycloalcoyle ayant de 3 à 12 atomes de carbone et jusqu'à 8 chaînons dans le cycle,

$R^5$ et $R^6$ sont identiques ou différents et représentent l'hydrogène, un groupe alcoyle ayant de 1 à 6 atomes de carbone, cycloalcoyle ayant de 3 à 10 atomes de carbone et jusqu'à 8 chaînons dans le cycle ou le radical $-[CH_2]_o-$ Ar dans lequel o est 1 ou 2, la chaîne $-[CH_2]_o-$ peut être substituée par 1 ou 2 groupes alcoyle en $C_1$ ou $C_2$ et Ar est un système aromatique ou hétéro-aromatique à 5 ou 6 chaînons, éventuellement substitué par de 1 à 3 groupes alcoyle en $C_1$ ou $C_2$, alcoxy en $C_1$ ou $C_2$, ou halogènes identiques ou différents, et

n est 1 ou 2,

les radicaux $R^5$ et $R^6$ et/ou deux des radicaux $R^2$, $R^3$ et $R^4$ pouvant également former une chaîne $-[CH_2]_m-$ avec m = 3 à 6, qui peut être éventuellement substituée par 1 ou 2 groupes méthyle et qui, dans le cas de $R^5$ et $R^6$, peut être également interrompue par 1 ou 2 atomes d'oxygène, de soufre et/ou groupes imino, ainsi que de leurs sels physiologiquement acceptables, caractérisé en ce qu'il consiste :

a) à faire réagir des composés de formule II

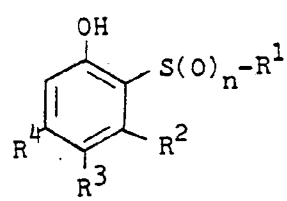

(II)

dans laquelle n, $R^1$, $R^2$, $R^3$ et $R^4$ ont les significations indiquées ci-dessus, avec un N-hydroxyméthylcarboxamide de formule générale III

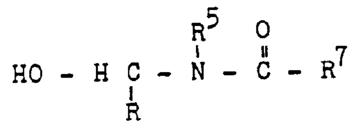

(III)

dans laquelle R a la signification précédente, $R^5$ représente l'hydrogène ou un groupe alcoyle ayant de 1 à 4 atomes de carbone et $R^7$ représente l'hydrogène, un groupe alcoyle ayant de 1 à 4 atomes de carbone, éventuellement substitué par un halogène ou un groupe aryle ayant de 6 à 10 atomes de carbone, ou dans laquelle $R^5$ et le radical $COR^7$ représentent ensemble le radical o-phtaloyle, pour obtenir des composés de formule générale IV

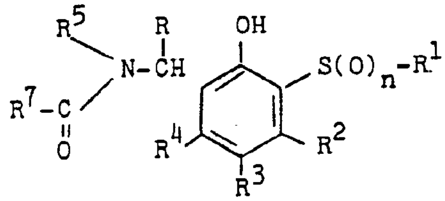

(IV)

et à éliminer le radical acyle $R^7$—CO par hydrolyse,

b) à faire réagir des composés de formule II dans laquelle les radicaux $R^1$ à $R^4$ et n ont les significations indiquées ci-dessus, en présence de formaldéhyde, avec des amines de formule générale V

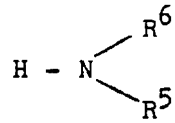
(V)

dans laquelle $R^5$ et $R^6$ ont les significations indiquées dans la revendication 1, à l'exception de l'hydrogène,

c) à faire réagir des composés de formule VI

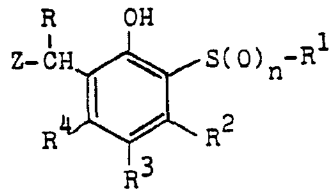
(VI)

dans laquelle n, R à $R^4$ ont les significations indiquées ci-dessus et Z représente un groupe labile, avec des amines de formule générale H—N = Y dans laquelle Y représente un groupe amino-protecteur ou les radicaux $R^5$ et $R^6$ définis dans la revendication 1, $R^6$ pouvant également être un groupe amino-protecteur, pour obtenir des composés de formule générale VII

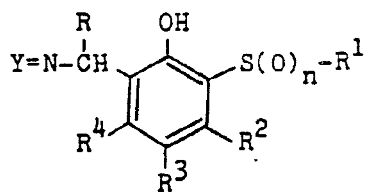
(VII)

et éventuellement à éliminer le groupe amino-protecteur par hydrolyse ou hydrogénolyse, ou

d) à faire réagir des composés de formule générale XIV

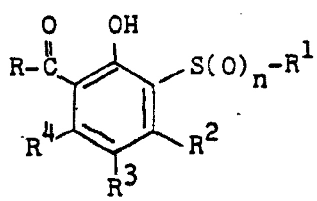
(XIV)

dans laquelle n et R à $R^4$ ont les significations indiquées ci-dessus, avec des amines de formule $R^5$—$NH_2$ avec $R^5$ ayant la signification ci-dessus, pour obtenir des bases de Schiff de formule XV

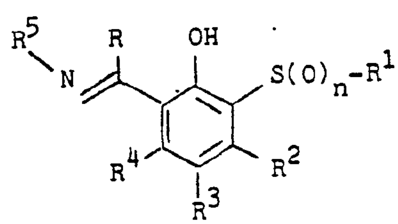
(XV)

dans laquelle n et R à $R^5$ ont les significations indiquées ci-dessus et à convertir ces bases en des composés de formule I, puis à convertir éventuellement les composés de formule I selon l'une des variantes a) à d) en leurs sels physiologiquement acceptables.

2. Procédé selon la revendication 1, caractérisé en ce que :

R représente l'hydrogène

$R^1$ représente un groupe alcoyle ayant de 1 à 4 atomes de carbone, cycloalcoyle ayant de 3 à 7 atomes de carbone et jusqu'à 6 chaînons dans le cycle, alcényle ayant de 2 à 6 atomes de carbone ou halogénoalcoyle ayant de 1 à 4 atomes de carbone et de 1 à 3 atomes d'halogène identiques ou différents,

$R^3$ représente un halogène, un groupe alcoyle ayant de 1 à 8 atomes de carbone ou cycloalcoyle ayant de 3 à 10 atomes de carbone et jusqu'à 7 chaînons dans le cycle.

$R^5$ et $R^6$ ont les significations définies dans la revendication 1, si ce n'est que $R^5$ et/ou $R^6$ ne représentent pas un groupe alcoyle ayant 1 ou 2 atomes de carbone,

n est 1 ou 2,

les radicaux $R^5$ et $R^6$ et/ou deux des radicaux $R^2$, $R^3$ et $R^4$ pouvant former une chaîne polyméthylène telle que définie dans la revendication 1.

3. Procédé selon la revendication 1, caractérisé en ce qu'on prépare le 2-aminométhyl-4-(1,1-diméthyléthyl)-6-méthylsulfonylphénol et on le convertit éventuellement en son sel d'addition avec un acide physiologiquement acceptable.

4. Procédé selon la revendication 1, caractérisé en ce qu'on prépare le 2-aminométhyl-4-(1,1-diméthyléthyl)-6-chlorométhylsulfonylphénol et qu'on le convertit éventuellement en son sel d'addition avec un acide, physiologiquement acceptable.

5. Procédé selon la revendication 1, caractérisé en ce qu'on prépare le 2-aminométhyl-4-(1,1-diméthylpropyl)-6-méthylsulfonylphénol et on le convertit éventuellement en son sel d'addition avec un acide, physiologiquement acceptable.

6. Procédé selon la revendication 1, caractérisé en ce qu'on prépare le méthyl-[3-aminométhyl-5-(1,1-diméthyl-éthyl)-2-hydroxyphényl] sulfoxyde et on le convertit éventuellement en son sel d'addition avec un acide, physiologiquement acceptable.

7. Procédé selon la revendication 1, caractérisé en ce qu'on prépare le 2-aminométhyl-4-isopropyl-6-méthylsulfonylphénol et on le convertit éventuellement en son sel d'addition avec un acide, physiologiquement acceptable.

8. Procédé selon la revendication 1, caractérisé en ce qu'on prépare le 2-aminométhyl-4-(1,1-diméthylpropyl)-6-éthylsulfonylphénol et on le convertit éventuellement en son sel d'addition avec un acide, physiologiquement acceptable.

9. Procédé selon la revendication 1, caractérisé en ce qu'on prépare le 2-aminométhyl-4-(1,1-diméthylpropyl)-6-iodométhyl-sulfonylphénol et on le convertit éventuellement en son sel d'addition avec un acide, physiologiquement acceptable.

10. Composition pharmaceutique contenant un composé selon la revendication 1.

11. Procédé de préparation d'une composition pharmaceutique selon la revendication 8, caractérisé en ce qu'on convertit un composé selon la revendication 1, éventuellement avec des véhicules et/ou stabilisants pharmacologiquement acceptables, en une forme d'administration appropriée.

12. Procédé de préparation de composés de formule générale II

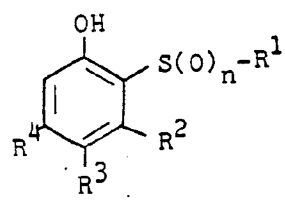

(II)

dans laquelle n, $R^1$ à $R^4$ ont les significations indiquées dans la revendication 1, caractérisé en ce qu'on oxyde un thioéther de formule VIII

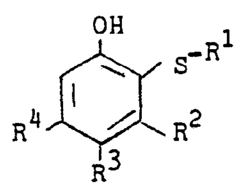

(VIII)

dans laquelle $R^1$ à $R^4$ ont les significations ci-dessus.